# EUROPÄISCHE PATENTSCHRIFT

(11) **EP 3 624 755 B1**
(45) Veröffentlichungstag und Bekanntmachung des Hinweises auf die Patenterteilung: **01.09.2021**
(21) Anmeldenummer: 18785661.2
(22) Anmeldetag: 18.10.2018
(51) Int. Cl.: A61K 8/02, A61K 8/26, A61K 8/55, A61K 8/81, A61K 8/19, A61K 8/34, A61K 8/36, A61K 8/37, A61K 8/58, A61K 8/72, A61Q 3/02, C09C 1/64

(54) **NAGELLACKKOMPOSITION ENTHALTEND GEPRÄGTE EFFEKTPIGMENTE SOWIE OBERFLÄCHENMODIFIZIERTE GEPRÄGTE EFFEKTPIGMENTE**
NAIL VARNISH COMPOSITION CONTAINING EMBOSSED EFFECT PIGMENTS AND SURFACE-MODIFIED EMBOSSED EFFECT PIGMENTS
COMPOSITION DU VERNIS À ONGLES COMPORTANT DES PIGMENTS À EFFET D'ESTAMPAGE AINSI QUE PIGMENTS À EFFET D'ESTAMPAGE MODIFIÉS EN SURFACE

(30) Priorität: 18.10.2017 EP 17001718
(43) Veröffentlichungstag der Anmeldung: 25.03.2020
(60) Teilanmeldung: 20197798.0
(73) Patentinhaber: ECKART GmbH, 91235 Hartenstein (DE)
(72) Erfinder: SCHILLING, Christine, 91235 Hartenstein (DE); GEBHARD, Ann-Katrin, 91235 Hartenstein (DE); SCHMIDT, Ulrich, 91235 Hartenstein (DE); STRUCK, Oliver, 91235 Hartenstein (DE)
(74) Vertreter: Altana IP Department
(86) Internationale Anmeldenummer: PCT/EP2018/078505
(87) Internationale Veröffentlichungsnummer: WO 2019/077021

(56) Entgegenhaltungen:
- EP-A1- 1 796 794
- WO-A1-2017/178610
- FR-A1- 2 939 678

## Beschreibung

Die vorliegende Erfindung betrifft ein oberflächenmodifiziertes Effektpigment sowie ein Verfahren zur Herstellung desselben sowie Nagellackkompositionen enthaltend dieses oberflächenmodifizierte Effektpigment.

WO 2005/055965 A1 offenbart kosmetische Zusammensetzungen wie Nagellacke, die geprägte Aluminiumpigmente mit Regenbogeneffekt enthalten. Nicht offenbart jedoch werden Bindemittel auf Basis von Kohlenwasserstoffharzen.

EP 1 462 085 A1 offenbart eine Nagellackkomposition mit Spiegeleffekt, welche Partikel mit metallischem Glanz in einem Anteil von ≥ 2 Gew.-%, bezogen auf das Gesamtgewicht der Nagellackkomposition, sowie Texturierungsmittel umfasst. EP 1 462 085 A1 offenbart keine oberflächenmodifizierten Effektpigmente. Weiterhin offenbart dieses Schutzrecht keine diffraktiven Effektpigmente.

EP 1 299 066 A2 beschreibt einen Aluminiumplättchen enthaltenden Nagellack mit spiegelartigem Aussehen. Gemäß EP 1 299 066 A2 muss der Nagellack als Filmbildner Nitrocellulose mit einem Molekulargewicht von > 56000 g/mol umfassen, damit ein spiegelartiger Effekt auf einem Fingernagel erzielt werden kann. Dieses Schutzrecht offenbart keine diffraktiven Effektpigmente.

EP 1 796 794 A1 offenbart eine kosmetische Zusammensetzung, welche ein PVD-Aluminiumpigment in einer Pigmentierungshöhe von 0,05 bis 5,0 Gew.-%, bezogen auf das Gesamtgewicht der kosmetischen Zusammensetzung, und wenigstens ein leafing-Additiv umfasst. Als leafing-Additiv dienen langkettige Phosphorsäureester oder ein Gemisch aus mehreren langkettigen Phosphorsäureestern. In den meisten Nagellacken jedoch kommt der leafing-Effekt nicht voll zur Geltung. Weiterhin offenbart dieses Schutzrecht keine diffraktiven Effektpigmente.

EP 2 248 514 A2 beschreibt nitrocellulosefreie Nagellackkompositionen, welche wenigstens ein Styrol/Maleinsäureanhydrid-Copolymer als hochglänzenden Filmbildner, wenigstens ein Epoxyharz als Co-Filmbildner, wenigstens eine reaktive Komponente sowie wenigstens ein Lösungsmittel umfasst. Die nitrocellulosefreie Nagellackkomposition soll vergleichbare oder bessere Haftungseigenschaften als nitrocellulosehaltige Nagellackkompositionen aufweisen. In EP 2 248 514 A2 werden als Farbmittel lediglich Perlglanzpigmente als Effektpigmente offenbart.

FR 2 939 678 A1 offenbart Nagellackkompositionen, die Bindemittel enthalten, welche einerseits ein Copolymer und andererseits ein Bindemittel auf Basis von Kohlenwasserstoffharzen, Acrylatharzen, Polyesterharzen, Polyamidharzen, Siliconharzen oder Mischungen davon enthalten. Als Pigmente werden jedoch neben konventionellen Pigmenten lediglich Perlglanzpigmente sowie als goniochromatische Pigmente Flüssigkristallpigmente oder Multilayerpigmente offenbart.

US 6,267,951 offenbart kosmetische Kompositionen für Anwendungen als Nagellack oder Haarfärbemittel, die ein Alkylcycloalkylacrylatcopolymer und einen Ester enthaltend Säuren, die vom Kolophonium abstammen, enthalten.

US 6692830 B2 offenbart in grundlegender Weise diffraktive Metalleffektpigmente mit Regenbogeneffekt, die auch mit diversen Interferenzschichten beschichtet sein können. Die Pigmente weisen verschiedene Liniendichten auf.

EP 1901870 B1 beschreibt die Herstellung diffraktiver Metalleffektpigmente mit einer mittleren Größe d₅₀ von über 75 µm.

EP 2598578 B1 offenbart dunkle geprägte Metalleffektpigmente.

Geprägte Metalleffektpigmente sind als solches bekannt und werden auch in Nagellacken eingesetzt. Jedoch besteht weiterhin Bedarf an einer Verbesserung des Regenbogeneffekts in Nagellacken.

WO 2017/178610 A1 offenbart Nagellackkompositionen enthaltend mit leafing-Additiven versetzte Effektpigmente auf Basis von Aluminium sowie Kohlenwasserstoffbasierende Bindemittel. Die Aluminiumeffektpigmente sind allerdings nicht geprägt, sondern weisen glatte Oberflächen auf.

Es hat sich gezeigt, dass die erfindungsgemäß oberflächenmodifizierten Effektpigmente nicht in allen Nagellacksystemen, wie sie zum Beispiel in der EP 1796794 B2 offenbart wurden, gute Ergebnisse zeigt. Häufig geht der Leafing-Effekt bei der Applikation oder danach verloren und damit einhergehend kommen die optischen Eigenschaften der Effektpigmente nicht vollkommen zur Geltung.

Aufgabe der vorliegenden Erfindung ist es, eine Nagellackkomposition enthaltend geprägte Effektpigmente bereitzustellen, welche einen verbesserten Regenbogeneffekt sowie ein verbessertes Chroma aufweist. Zudem sollte möglichst auch die Brillanz verbessert werden.

Eine weitere Aufgabe besteht darin, ein Verfahren zur Herstellung des erfindungsgemäßen Nagellacks bereit zu stellen.

Weiterhin soll ein Verfahren zur Beschichtung von kerartinhaltigen Substraten mit einem Nagellack bereit gestellt werden.

Die der Erfindung zugrunde liegende Aufgabe wird gelöst durch die Bereitstellung einer Nagellackkomposition enthaltend
a) ein geprägtes Effektpigment umfassend ein plättchenförmiges metallisches Substrat mit geprägter Struktur, welche ein periodisches Muster mit diffraktiven Elementen aufweist und welches durch PVD-Verfahren hergestellt wird und optional wenigstens eine auf dem Substrat aufgebrachte Beschichtung, wobei das Substrat einen Gehalt an elementarem Metall von 80 bis 100 Gew.-%; bezogen auf das Substrat aufweist und wobei das Effektpigment mit einem leafing-Additiv zur Oberflächenmodifizierung behandelt wurde,
b) wenigstens ein Kohlenwasserstoffharz als Bindemittel,
c) wenigstens ein Lösemittel oder Lösemittelgemisch und
d) optional weitere Hilfsstoffe.

Bevorzugte Ausführungsformen der erfindungsgemäßen Nagellackkomposition finden sich in den Unteransprüchen 2 bis 14.

Die Aufgabe der zugrunde liegende Erfindung wird ebenfalls gelöst durch ein Verfahren zur Herstellung der erfindungsgemäßen Nagellackkomposition umfassend die Schritte
i) Oberflächenmodifizierung des Effektpigments durch ein leafing-Additiv in einer Dispersion in einem Lösemittel,
ii) Lösen des Kohlenwasserstoffharz in einem Lösemittel oder Lösemittelgemisch
iii) Vermischen und Homogenisieren der Dispersion nach i) mit der Bindemittellösung nach ii), sowie
iv) gegebenenfalls Ergänzen mit weiterem Lösemittel oder Lösemittelgemisch.

Eine bevorzugte Ausführungsform dieses Verfahrens findet sich in Unteranspruch 15.

Weiterhin wird ein Verfahren zur Beschichtung eines natürlichen oder künstlichen Fingernagels beschrieben umfassend die Schritte:
a) Beschichtung des natürlichen oder künstlichen Fingernagels mit einer Nagellackkomposition gemäß der Ansprüche 1 bis 14 und anschließendem Trocknen des Nagellacks,
b) optional darauffolgende Beschichtung des Nagellacks mit einem Klarlack.

### Erfindungsgemäße Nagellackkomposition:

Die Erfindung richtet sich auf eine Nagellackkomposition, die den leafing-Effekt der Effektpigmente in ausgezeichneter Weise ermöglicht und erhält.

Diese erfindungsgemäße Nagellackkomposition enthält:
a) ein Effektpigment umfassend ein plättchenförmiges metallisches Substrat mit geprägter Struktur, welche ein periodisches Muster mit diffraktiven Elementen aufweist und welches durch PVD-Verfahren hergestellt wird und optional wenigstens eine auf dem Substrat aufgebrachte Beschichtung, wobei das Substrat einen Gehalt an elementarem Metall von 80 bis 100 Gew.-%; bezogen auf das Substrat aufweist und wobei das Effektpigment mit einem leafing-Additiv zur Oberflächenmodifizierung behandelt wurde,
b) wenigstens ein Kohlenwasserstoffharz als Bindemittel,
c) wenigstens ein Lösemittel oder Lösemittelgemisch und
d) optional weitere Hilfsstoffe.

Die erfindungsgemäße Nagellackkomposition, welche wenigstens ein oberflächenmodifiziertes Effektpigment umfasst, weist im Unterschied zu den meisten kommerziell erhältlichen Nagellackkompositionen vorzugsweise keine Nitrocellulose und kein Celluloseacetatbutyrat auf. Das optische Erscheinungsbild der erfindungsgemäßen Nagellackkomposition wird, nach Applikation und Trocknung, maßgeblich durch das wenigstens eine oberflächenmodifizierte geprägte Effektpigment bestimmt.

Die erfindungsgemäße Nagellackkomposition umfasst das wenigstens eine oberflächenmodifizierte geprägte Effektpigment bevorzugt in einem Anteil aus einem Bereich von 0,2 Gew.-% bis 7,0 Gew.-%, weiter bevorzugt aus einem Bereich von 0,3 Gew.-% bis 5,0 Gew.-%, besonders bevorzugt aus einem Bereich von 0,35 Gew.-% bis 3,0 Gew.-% und ganz besonders bevorzugt aus einem Bereich von 0,4 Gew.-% bis 2,0 Gew.-%, jeweils bezogen auf das Gesamtgewicht der Nagellackkomposition.

In den erfindungsgemäßen Nagellackkompositionen ordnen sich die oberflächenmodifizierten geprägten Effektpigmente bevorzugt an der Oberfläche aufgebrachten Nagellacks an. Unter "an der Oberfläche anordnen" wird erfindungsgemäß verstanden, dass sich die oberflächenmodifizierten Effektpigmente auf der Nagellackbasis und/oder ausgehend von der Grenzfläche Nagellackkomposition/Luft bzw. Nagellackkomposition/Überlackierung in Richtung des lackierten Untergrunds im an diese Grenzfläche anschließenden Drittel der Nagellackkomposition befinden. Bevorzugt schwimmen die oberflächenmodifizierten Effektpigmente im Nagellack auf und richten sich an der Nagellackoberfläche aus. Die oberflächenmodifizierten Effektpigmente zeichnen sich mithin in der erfindungsgemäßen Nagellackkomposition durch ein ausgeprägtes leafing-Verhalten aus.

Aufgrund dieses ausgeprägten leafing-Verhaltens der oberflächenmodifizierten Effektpigmente lassen sich erfindungsgemäß Nagellackkompositionen herstellen, welche ihr optisches Erscheinungsbild hauptsächlich dem der Nagellackbasis zugesetzten wenigstens einem oberflächenmodifizierten Effektpigment verdanken. Es kommt daher der Regenbogeneffekt und auch das Chroma der geprägten Effektpigmente weit besser zur Geltung.

In weiteren Ausführungsformen können der Nagellackkomposition je nach zu erzielendem optischen Effekt auch weitere oberflächenmodifizierte, vorzugsweise nicht-beugende, Effektpigmente zugesetzt werden, wobei hierbei sowohl die Oberflächenmodifizierung als auch die Effektpigmente verschieden voneinander sein können. Ebenfalls können der erfindungsgemäßen Nagellackkomposition konventionelle organische und/oder anorganische Pigmente zugesetzt werden.

### Geprägte Effektpigmente:

Erfindungsgemäß enthält die erfindungsgemäße Nagellackkomposition ein Effektpigment umfassend ein plättchenförmiges metallisches Substrat mit geprägter Struktur, welche ein periodisches Muster mit diffraktiven Elementen aufweist, welches durch PVD-Verfahren hergestellt wird und eine Liniendichte von 5.000 bis 20.000 Linien/cm aufweist, und optional wenigstens eine auf dem Substrat aufgebrachte Beschichtung, wobei das Substrat einen Gehalt an elementarem Metall von 80 bis 100 Gew.-%; bezogen auf das Substrat aufweist.

Die geprägte Struktur weist erfindungsgemäß ein periodisches Muster mit diffraktiven Elementen auf. Hierbei bezieht sich das periodische Muster auf die kleinste Einheit der diffraktiven Elemente. Vorzugsweise weist die periodische diffraktive Struktur 5.000 bis 20.000 diffraktive Elemente/cm auf, besonders bevorzugt 9.000 - 18.000 diffraktive Elemente/cm und ganz besonders bevorzugt 12.000 - 16.000 diffraktive Elemente/cm auf. Innerhalb dieses Bereiches wird vorwiegend sichtbares Licht (ca. 400 bis 800 nm Wellenlänge) gemäß dem bekannten Prinzip eines Beugungsgitters an den diffraktiven Elementen gebeugt, wodurch der Betrachter einen Regenbogeneffekt wahrnimmt. Es können jedoch auch Anteile der IR-Strahlung und/oder der UV-Strahlung gebeugt werden.

Die Periodizität bestimmt wesentlich die gebeugten Wellenlängen des einfallenden Lichts. Im Einzelnen kann dies nach bekannten Formeln berechnet werden, wie sie beispielsweise der US 6,692,830 B2 zu entnehmen sind.

Als diffraktive Elemente kommen beispielsweise symmetrische Dreiecke, asymmetrische Dreiecke, Rillen unterschiedlichster Formen, Rechteckfunktionen, Kreise, wellenförmige Linien, Kegel, Kegelstümpfe, Noppen, Prismen, Pyramiden, Pyramidenstümpfe, Zylinder, Halbkugeln, etc. sowie Kombinationen dieser geometrischen Formen und Körper in Betracht.

Geometrische Körper mit einer oder mehreren zur Pigmentoberfläche parallel angeordneten Flächen, wie beispielsweise Kegelstümpfe, Pyramidenstümpfe, Zylinder oder Rechteckfunktionen weisen aufgrund dieser Flächen ein höheres Reflexionsvermögen auf.

Geometrische Körper, die, bezogen auf die Pigmentoberfläche, schräge Seitenoberflächen aufweisen, verstärken den Regenbogeneffekt. Unter schrägen Seitenflächen werden Seitenflächen, die, bezogen auf den Untergrund, einen Winkel von 5 bis 89°, vorzugsweise von 15 bis 84°, noch weiter bevorzugt von 27 bis 80°, noch weiter bevorzugt von 43 bis 74°, aufweisen, verstanden. Geeignete geometrische Körper sind beispielsweise Kegel, Kegelstümpfe, Pyramiden, Pyramidenstümpfe, etc.

Bei Kegelstümpfen erfolgt beispielsweise sowohl eine Reflexion an der zur Pigmentoberfläche parallelen Deckfläche und eine Verstärkung des Regenbogeneffektes an der Mantelfläche. Entsprechend erfolgt bei Pyramidenstümpfen die Reflexion an der Deckfläche und eine Verstärkung des Regenbogeneffektes an den schrägen Seitenflächen.

Selbstverständlich ist es auch möglich, bei Kegelstümpfen oder Pyramidenstümpfen die Deckfläche nicht parallel zur Pigmentoberfläche, sondern schräg zur Pigmentoberfläche anzuordnen.

Über die durch Prägung und/oder Formung auf der Oberfläche des plättchenförmigen metallischen Substrats angeordneten geometrischen Körper kann der Regenbogeneffekt und/oder das Reflexionsvermögen des Effektpigments verstärkt oder das relative Verhältnis von Regenbogeneffekt zu Reflexion geändert werden.

So können die geometrischen Körper abschnittsweise getrennt oder auch miteinander gemischt vorliegen. Auch ist es selbstverständlich möglich, die geometrischen Formen und geometrischen Körper überlagert anzuordnen, so dass beispielsweise auf einer wellenförmigen Struktur zusätzlich geometrische Körper angeordnet sind.

Gemäß einer weiteren Variante der Erfindung können auf der plättchenförmigen metallischen Substratoberfläche ungeprägte bzw. ungeformte, mithin glatte Abschnitte neben geprägten und/oder geformten Abschnitten vorliegen. Auch hierdurch kann das relative Verhältnis von Regenbogeneffekt zu Reflexion geändert werden.

Bevorzugt ist die gesamte Oberfläche des plättchenförmigen metallischen Substrats mit der diffraktiven Struktur versehen, vorzugsweise geprägt. Es kann jedoch auch nur ein Teil der Metalleffektpigmentoberfläche mit der diffraktiven Struktur versehen, vorzugsweise geprägt, werden bzw. zu einer diffraktiven Struktur ausgeformt sein. Bevorzugt werden mindestens 60 %, besonders bevorzugt mindestens 75% und ganz besonders bevorzugt mindestens 90% der Metalleffektpigmentoberfläche mit einer diffraktiven Struktur geprägt bzw. zu einer diffraktiven Struktur ausgeformt sein.

Bei einer besonders bevorzugten Ausführungsform umfasst oder besteht die diffraktive Struktur aus wellenförmigen, beispielsweise sinusförmigen, Linien, Kegeln oder Kegelstümpfen. Bei einer ganz besonders bevorzugten Ausführungsform umfasst oder besteht die diffraktive Struktur aus sinusförmigen Linien, da diese Form zum einen besonders leicht einzuprägen ist und zum anderen einen sehr starken Beugungseffekt bewirkt. Bei diesen sinusförmigen Linien ist die diffraktive Struktur vorzugsweise auf dem ganzen plättchenförmigen metallischen Substrat eingeprägt.

Gemäß einer bevorzugten Ausführungsform enthält die erfindungsgemäße Nagellackkomposition ein Effektpigment umfassend ein plättchenförmiges, metallisches Substrat mit geprägter Struktur, wobei das Metall aus der Gruppe bestehend aus Aluminium, Kupfer, Chrom, Eisen oder Legierungen hiervon, entnommen wird.

Besonders bevorzugt enthält das plättchenförmige, metallische Substrat mit geprägter Struktur ein Metall aus Aluminium oder besteht daraus, welches besonders gut reflektiert und leicht durch PVD-Verfahren herzustellen ist.

Um einen deutlich wahrnehmbaren Effekt erzielen zu können, weist die diffraktive Struktur, bevorzugt Linienstruktur, vorzugsweise eine gewisse Mindesttiefe auf, da ansonsten der physikalische Effekt der Beugung nur unzureichend ausgebildet sein kann. Daher sollte die diffraktive Struktur bevorzugt eine Tiefe (gemessen als "Berg zu Tal" gemäß der WO 2005/055965 A1) von mindestens 40 nm, bevorzugt 40 nm bis 600 nm und besonders bevorzugt von 50 nm bis 400 nm und ganz besonders bevorzugt von 100 nm bis 250 nm aufweisen.

Oberhalb von 600 nm kann die Stabilität der Struktur als Ganzes nicht mehr gegeben sein. Unterhalb von 40 nm ist der diffraktive Effekt zu gering ausgeprägt.

Die plättchenförmigen metallischen geprägten Substrate werden durch PVD-Verfahren hergestellt.

Bevorzugt weist das metallische plättchenförmige, eingravierte Substrat eine mittlere Dicke h₅₀ (Medianwert) aus einem Bereich von 20 nm bis 80 nm besonders bevorzugt aus einem Bereich von 30 bis 60 nm auf.

Die Bestimmung des Medianwertes h₅₀ erfolgte bevorzugt gemäß der in der WO 2004/087816 A2 (Seiten 24 und 25) beschriebenen Methode mittels REM.

Unterhalb von 20 nm wird das Substrat möglicherweise zu dunkel und kann die nötige mechanische Festigkeit, die zum Erhalt der eingeprägten Struktur nötig ist, verlieren.

Oberhalb von 80 nm wird das metallische Substrat zu dick, um die nötige Brillanz und Deckkraft hervorzurufen.

Die Tiefe der diffraktiven Struktur kann mithin die mittlere Schichtdicke h₅₀ des plättchenförmigen metallischen Substrats übertreffen.

Die mittlere Größe d₅₀ (Median) der Effektpigmente mit geprägter Struktur liegt in einem Bereich von 5 - 120 µm, bevorzugt in einem Bereich von 10- 75 µm und ganz besonders bevorzugt in einem Bereich von 15 - 40 µm.
Hierbei wird der d₅₀-Wert mittels Laserbeugungsmethoden als volumengemittelte Summendurchgangskurve der Größenverteilung (Fraunhofer -Beugung) in für den Fachmann üblichen Weise bestimmt. Hierbei wird als Messgerät das Gerät Horiba LA-950 der Fa. Horiba verwendet.

Der h₅₀-Wert der Summenhäufigkeitsverteilung der Größenverteilungsfunktion gibt an, dass 50% der vermessenen Effektpigmente eine Größe aufweisen, der gleich oder kleiner als der jeweils angegebene Wert ist.

Unterhalb von 5 µm ist die Anzahl der diffraktiven Strukturen pro Pigmentteilchen zu gering, um einen wirkungsvollen Regenbogeneffekt hervorzurufen. Oberhalb von 120 µm ist das Effektpigment zu groß, um sich in der Nagellackformulierung nach dem Auftragen auf einen künstlichen oder natürlichen Finger ausreichend planparallel zum Fingeruntergrund zu orientieren. Bei einer schlechten Orientierung wird ebenfalls der Regenbogeneffekt sowie das Chroma gestört aufgrund einer sich teilweise auslöschenden Überlagerung des von unterschiedlich orientierten Effektpigmentteilchen abgestrahlten gebeugten Lichtes.

Werden Metalleffektpigmente in kosmetischen Formulierungen eingesetzt, so müssen sie gewissen Reinheitsanforderungen genügen wie beispielsweise die EU Cosmetic Regulation 1223/2009 oder FDA 21CFR part 73.
Werden beispielsweise Aluminiumplättchen als metallisches plättchenförmiges Substrat eingesetzt, weisen diese bevorzugt einen Aluminiumgehalt von ≥ 97 Gew.-%, weiter bevorzugt von ≥ 98 Gew.-%, besonders bevorzugt von ≥ 99 Gew.-% und ganz besonders bevorzugt von ≥ 99,7 Gew.-%, jeweils bezogen auf das Gesamtgewicht des Aluminiumplättchens, auf. Bei einer bevorzugten Ausführungsform weisen die Aluminiumplättchen weiterhin einen Quecksilbergehalt von bevorzugt ≤ 1 ppm, einen Arsengehalt von bevorzugt ≤ 2 ppm, einen Bleigehalt von bevorzugt ≤ 10 ppm, einen Cadmiumgehalt von bevorzugt ≤ 1 ppm, einen Bariumgehalt von bevorzugt ≤ 10 ppm, einen Chromgehalt von bevorzugt ≤ 20 ppm, einen Nickelgehalt von bevorzugt ≤ 20 ppm, einen Kupfergehalt von bevorzugt ≤ 20 ppm, einen Cobaltgehalt von bevorzugt ≤ 20 ppm, einen Antimongehalt von bevorzugt ≤ 2 ppm, einen Selengehalt von bevorzugt ≤ 10 ppm und einen Zinkgehalt von bevorzugt ≤ 20 ppm auf.
Insbesondere ist bevorzugt, dass die Aluminiumplättchen einen Quecksilbergehalt von bevorzugt ≤ 1 ppm, einen Arsengehalt von bevorzugt ≤ 2 ppm, einen Bleigehalt von bevorzugt ≤ 10 ppm und einen Cadmiumgehalt von bevorzugt ≤ 1 ppm aufweisen.

Werden Kupferplättchen als metallisches plättchenförmiges Substrat eingesetzt, weisen diese bevorzugt einen Kupfergehalt von ≥ 95 Gew.-%, weiter bevorzugt von ≥ 96 Gew.-%, besonders bevorzugt von ≥ 97 Gew.-% und ganz besonders bevorzugt von ≥ 98 Gew.-%, jeweils bezogen auf das Gesamtgewicht des Kupferplättchens, auf. Bei einer bevorzugten Ausführungsform weisen die Kupferplättchen weiterhin einen Quecksilbergehalt bevorzugt von ≤ 1 ppm, einen Arsengehalt bevorzugt von ≤ 3 ppm, einen Bleigehalt bevorzugt von ≤ 20 ppm, einen Cadmiumgehalt bevorzugt von ≤ 15 ppm, einen Bariumgehalt bevorzugt von ≤ 10 ppm, einen Chromgehalt bevorzugt von ≤ 20 ppm, einen Nickelgehalt bevorzugt von ≤ 20 ppm, einen Cobaltgehalt bevorzugt von ≤ 20 ppm, einen Antimongehalt bevorzugt von ≤ 2 ppm und einen Selengehalt bevorzugt von ≤ 10 ppm auf.

Werden Goldbronzeplättchen als metallisches plättchenförmiges Substrat eingesetzt, weisen diese bevorzugt einen Kupfergehalt aus einem Bereich von 70 Gew.-% bis 95 Gew.-%, einen Zinkgehalt aus einem Bereich von < 5 Gew.-% bis < 30 Gew.-%, einen Aluminiumgehalt aus einem Bereich von 0,01 Gew.-% bis ≤ 1,5 Gew.-%, einen Zinngehalt aus einem Bereich von 0,001 Gew.-% bis ≤ 0,5 Gew.-%, jeweils bezogen auf das Gesamtgewicht der Goldbronzeplättchen, auf. Bei einer bevorzugten Ausführungsform weisen die Goldbronzeplättchen weiterhin einen Quecksilbergehalt bevorzugt von ≤ 1 ppm, einen Arsengehalt bevorzugt von ≤ 3 ppm, einen Bleigehalt bevorzugt von ≤ 20 ppm, einen Cadmiumgehalt bevorzugt von ≤ 15 ppm, einen Bariumgehalt bevorzugt von ≤ 10 ppm, einen Chromgehalt bevorzugt von ≤ 20 ppm, einen Nickelgehalt bevorzugt von ≤ 20 ppm, einen Cobaltgehalt bevorzugt von ≤ 20 ppm, einen Antimongehalt bevorzugt von ≤ 2 ppm und einen Selengehalt bevorzugt von ≤ 10 ppm auf.

Gemäß einer Ausführungsform enthalt die erfindungsgemäße Nagellackkomposition als Effektpigment ein metallisches plättchenförmiges, metallisches eingraviertes Substrat mit diffraktiven Elementen, welches keine weiteren optisch aktiven Beschichtungen aufweist. Bevorzugt ist dieses Substrat aus Aluminium.

Gemäß einer weiterhin bevorzugten Ausführungsform sind die erfindungsgemäß zu verwendenden metallischen, plättchenförmige geprägte Substrate, abgesehen von der erfindungsgemäß notwendigen Beschichtung mit einem leafing-Additiv, nicht weiter beschichtet. Hierbei werden sich natürlich an Luft ausbildende Metalloxidschichten wie beispielweise eine Aluminiumoxidschicht, nicht als Beschichtung angesehen.

In diesem Fall besteht das Effektpigment nur aus einer metallischen Schicht, in die die geprägte Struktur, vorzugsweise eine Linienstruktur, eingeprägt ist. Derartige Effektpigmente sind besonders bevorzugt, da ihre Gesamtdicke aufgrund des Fehlens weiterer Beschichtungen, minimal ist. Derartige Pigmente lassen sich besonders gut in Nagellacke einarbeiten.

Die Herstellung derartiger Effektpigmente ist beispielsweise in der EP 643745 B1 oder der EP 1901870 B1 beschrieben.
Kommerziell erhältlich sind derartige Pigmente beispielsweise unter den Handelsnamen Metalure® Prismatic und Silverdream Prismatic der Fa. Eckart America.

In einer weiteren erfindungsgemäßen Ausführungsform weist das metallische plättchenförmige, eingravierte Substrat, bevorzugt ein Aluminiumsubstrat, optisch aktive Beschichtungen enthaltend mindestens ein Schichtpaket aus:
A) einer niedrigbrechenden Schicht mit einem Brechungsindex < 1,8 und
B) einer hochbrechenden Schicht mit einem Brechungsindex von über 2,0 auf.
Bevorzugt werden auch diese Schichten mittels PVD-Verfahren hergestellt.

Die niedrigbrechende Schicht besteht bevorzugt aus SiO₂, Al₂O₃, B₂O₃ oder MgF₂.

Die bei dieser Ausführungsform vorhandene hochbrechende Beschichtung kann wenigstens eine hochbrechende Schicht aus oder mit wenigstens einem Metalloxid, Metallhydroxid und/oder Metalloxidhydrat umfassen, wobei das Metallion bevorzugt aus der Gruppe der Metalle, bestehend aus Ti, Fe, Sn, Mn, Zr, Sr, Ba, Ni, Ag, Zn, Cu, Cr und Co, ausgewählt ist, und weiter bevorzugt ausgewählt aus der Gruppe der Metalle, bestehend aus Ti, Fe, Sn, Zr, Zn, und Cr, und besonders bevorzugt ausgewählt aus der Gruppe der Metalle, bestehend aus Ti, und Fe ist.

In weiteren Ausführungsformen können auch hochbrechende Metallsulfide wie beispielsweise MoS₂ verwendet werden.

Bei einer weiteren Ausführungsform kann die optional vorhandene hochbrechende Beschichtung alternativ oder zusätzlich zu der wenigstens ein Metalloxid, Metallhydroxid und/oder Metalloxidhydrat umfassenden Schicht wenigstens eine semitransparente Metallschicht umfassen. Die Metalle der semitransparenten Metallschicht können aus der Gruppe, bestehend aus Ag, Al, Cr, Ni, Au, Pt, Pd, Cu, Zn und Ti, bevorzugt ausgewählt aus der Gruppe, bestehend aus Ag, Au und Cu, ausgewählt sein. Selbstverständlich kann die semitransparente Metallschicht auch Legierungen oder Mischungen der vorstehend aufgeführten Metalle umfassen. Die mittlere Dicke der semitransparenten Metallschicht liegt bevorzugt in einem Bereich von 1 nm bis 30 nm, besonders bevorzugt in einem Bereich von 4 nm bis 26 nm und besonders bevorzugt in einem Bereich von 7 nm bis 21 nm.

In weiteren Ausführungsformen weist das metallische plättchenförmige, eingravierte Substrat, bevorzugt ein Aluminiumsubstrat, lediglich eine beidseitige durch PVD-Verfahren aufgebrachte Schicht auf, die bevorzugt niedrig brechend ist. Derartige Schichten sollen nicht zur optischen Aktivität des Effektpigments beitragen, sondern diesem lediglich eine mechanische Stabilität verleihen. Derartige Effektpigmente sind beispielsweise in der WO 2000/34395 beschrieben.

So werden beispielsweise von der Firma Viavi (Santa Rosa, Kalifornien, USA) unter dem Handelsnamen SpectraFlair® geprägte Effektpigmente mit entsprechenden Beschichtungen angeboten.

Weitere Beispiele derartiger Effektpigmente weisen folgende Schichtstrukturen auf.
- Al/SiO₂/Al/SiO₂/Al
- Cr/MgF₂/Al/MgF₂/Cr
- MoS₂/SiO₂/Al/SiO₂/MoS₂
- Fe₂O₃/SiO₂/Al/SiO₂/Fe₂O₃

Die Herstellung derartiger Effektpigmente ist beispielsweise in der US 6,749,777 B2 beschrieben.
Bei diesen Effektpigmenten wird der Farbeindruck neben der Beugung durch die diffraktive Struktur noch zusätzlich durch Interferenzphänomene der zusätzlichen Beschichtungen beeinflusst. Hierdurch kann sich eine "Störung" des reinen Regenbogeneffekts ergeben. Im Rahmen dieser Erfindung wird der erhaltene Effekt dennoch auch mit "Regenbogeneffekt" beschrieben.

Bei einer weiteren Ausführungsform besteht das plättchenförmige, metallische geprägte Substrat aus einer Mischschicht aus Metall, welches vorzugsweise überwiegend als Nanometall vorliegt und einem Metalloxid, wobei das Metall und das Metalloxid dasselbe Metall aufweisen. Derartige PVD-Effektpigmente sind in der EP 2598578 B1 beschrieben und sie zeichnen sich dadurch aus, dass die Reflektion dieser Pigmente sehr gering ist. Es handelt sich praktisch um schwarze Effektpigmente, die jedoch aufgrund ihrer Prägung dennoch einen Regenbogeneffekt aufweisen. Als Metall sind hier vorzugsweise Aluminium oder Chrom vorgesehen. Die mittleren Dicken derartiger geprägten Effektpigmente liegen in einem Bereich von 40 bis 130 nm.

Bei einer weiteren Ausführungsform weisen die erfindungsgemäßen, in einer Nagellackkomposition zu verwendenden, oberflächenmodifizierten Effektpigmente basierend auf metallischen plättchenförmigen Substraten mit Beschichtungen bevorzugt eine mittlere Gesamtdicke h₅₀ aus einem Bereich von 20 nm bis 4000 nm, weiter bevorzugt aus einem Bereich von 30 nm bis 3000 nm, besonders bevorzugt aus einem Bereich von 70 nm bis 2000 nm und ganz besonders bevorzugt aus einem Bereich von 230 nm bis 1300 nm auf. Unter mittlerer Gesamtdicke wird die komplette mittlere Dicke des oberflächenmodifzierten Effektpigments, also metallisches plättchenförmiges Substrat plus optionale Beschichtung plus Oberflächenmodifizierung, verstanden.

### Leafing-Additiv:

Es ist ein wesentlicher Bestandteil der vorliegenden Erfindung die Bereitstellung eines Nagellacks, in dem die leafing-Eigenschaften der oberflächenmodifizierten Effektpigmente sehr gut zur Geltung kommen.

Als leafing-Additiv zur Oberflächenmodifizierung des plättchenförmigen, metallischen Substrats wird bevorzugt wenigstens eine Verbindung aus der Gruppe bestehend aus phosphorsäureesterhaltigen, phosphonsäurehaltigen, fettsäurehaltigen und/oder silanhaltigen Verbindungen oder Mischungen hiervon, entnommen.

Es werden in einer bevorzugten Ausführungsform bei den zur Oberflächenmodifizierung einsetzbaren leafing-Additiven die der Gruppe bestehend aus Phosphorsäureestern der allgemeinen Formel:

(R-O)ₓ-P(O)(OR¹)₍₃₋ₓ₎ (I)

und/oder aus Phosphonsäureestern und/oder Phosphonsäuren der allgemeinen Formel

R-P(O)(OR²)(OR³) (II)

verwendet. Hierbei ist x = 1 bis 3 und die Reste R, R¹, R² und R³ haben folgende Bedeutung:
R = linearer und/oder verzweigter Alkylrest mit einer Kohlenstoffkette aus einem Bereich von C₈ bis C₂₀ und
R¹ = H, linearer und/oder verzweigter Alkylrest mit einer Kohlenstoffkette aus einem Bereich von C₁ bis C₆, bevorzugt C₁ bis C₃, wobei R¹ bei x = 1 identisch oder verschieden voneinander sein kann, und
R² = R³ = H, linearer und/oder verzweigter Alkylrest mit einer Kohlenstoffkette aus einem Bereich von C₁ bis C₆, bevorzugt C₁ bis C₃, wobei R² und R³ identisch oder verschieden voneinander sein können.

Bei den Phosphorsäureestern gemäß Formel (I) ist x in bevorzugten Ausführungsformen 1 oder 2, wobei auch Mischungen zwischen dem Mono- und Diphosphorsäureester möglich sind. R ist weiter bevorzugt C₁₀ bis C₂₀ und besonders bevorzugt C₁₂ bis C₁₈ sowie ganz besonders bevorzugt C₁₂ bis C₁₆. In weiter bevorzugten Ausführungsformen ist R = C₁₂ bis C₁₈ und R¹ = H.

In weiter bevorzugten Ausführungsformen werden als Additiv zur Oberflächenmodifizierung des geprägten Effektpigments Phosphonsäure, Phosphonsäureester und Phosphorsäureester der Gruppe Laurylphosphonsäuremethylester, Laurylphosphonsäureethylester Laurylphosphonsäure (R = 12) oder Monocetylphosphorsäureester, Dicetylphosphorsäureester (R = 16) und Mischungen hiervon verwendet.

Ganz besonders bevorzugt wird als leafing-Additiv Monocetylphosphorsäureester. Dicetylphosphorsäureester (R = 16) und Mischungen hiervon verwendet.

Bei den zur Oberflächenmodifizierung einsetzbaren Fettsäuren kann es sich um Fettsäuren der allgemeinen Formel

R'-COOH (III)

handeln, wobei der Rest R' ein linearer oder verzweigter Alkylrest mit einer Kohlenstoffkette aus einem Bereich von C₁₂ bis C₂₆, bevorzugt aus einem Bereich von C₁₄ bis C₂₄, weiter bevorzugt aus einem Bereich von C₁₆ bis C₂₂ und besonders bevorzugt aus einem Bereich von C₁₈ bis C₂₀ ist.

Bei einer bevorzugten Ausführungsform werden zur Oberflächenmodifizierung der in einer erfindungsgemäßen Nagellackkomposition einsetzbaren Effektpigmente Fettsäuren mit R' ein linearer, unsubstituierter Alkylrest mit einer Kohlenstoffkette aus einem Bereich von C₁₂ bis C₂₀, vorzugsweise aus einem Bereich von C₁₄ bis C₁₈ eingesetzt.

Da die Effektpigmente durch PVD-Verfahren hergestellt werden, enthalten sie im Gegensatz zu durch Vermahlung erhaltene Metalleffektpigmente keine Fettsäuren. Daher müssen auch die Fettsäuren als Oberflächenmodifizierung auf die geprägten Effektpigmente aufgebracht werden.

Bei den zur Oberflächenmodifizierung einsetzbaren Silanen kann es sich um Silane der allgemeinen Formel

R"-Si(OR⁴)₃ (IV)

handeln, wobei die Reste R" und R⁴ bevorzugt folgende Bedeutung haben: R" = linearer und/oder verzweigter Alkylrest mit einer Kohlenstoffkette aus einem Bereich von C₈ bis C₂₄, bevorzugt aus einem Bereich von C₁₀ bis C₂₂, besonders bevorzugt aus einem Bereich von C₁₂ bis C₁₈ und R⁴ = linearer und/oder verzweigter Alkylrest mit einer Kohlenstoffkette aus einem Bereich von C₁ bis C₄, bevorzugt aus einem Bereich von C₁ bis C₃ und besonders bevorzugt aus einem Bereich von C₁ bis C₂. In weiteren Ausführungsformen kann der Alkylrest R" in wenigstens einen Substituenten, ausgewählt aus der Gruppe, bestehend aus -OH, -OCH₃, -OC₂H₅, -NH₂, linearer und/oder verzweigter Alkylrest mit einer Kohlenstoffkette aus einem Bereich von C₁ bis C₆ umfassen.

Es ist besonders bevorzugt, dass die geprägten Effektpigmente in einem separaten Schritt mit dem Additiv beschichtet werden, bevor sie in das Nagellacksystem eingetragen werden.

Ein erfindungsgemäßes Verfahren zur Oberflächenmodifizierung der geprägten Effektpigmente umfasst die folgenden Schritte:
i. Suspendieren des geprägten Effektpigments umfassend ein plättchenförmiges metallisches Substrat mit geprägter Struktur in wenigstens einem Lösemittel,
ii. Zugabe des leafing-Additivs bei optional erhöhter Temperatur zu der Suspension aus Schritt i. und Rühren der nun erhaltenen Suspension,
iii. Abtrennen und optional Trocknen, des gemäß Schritt ii. erhaltenen oberflächenmodifizierten Effektpigments vom Lösemittel.

Als Lösemittel werden hierbei Lösemittel verwendet, die einerseits möglichst kompatibel und mit dem erfindungsgemäßen Nagellacksystem und physiologisch verträglich sind und andererseits das leafing-Additiv hinreichend zu lösen vermögen.

Bei Verwendung von Phosphorsäureestern, Phosphonsäureestern, Phosphonsäuren oder Fettsäuren als leafing-Additive sind bevorzugte Lösemittel sind Ethylacetat und Butylacetat und deren Mischungen und ganz besonders bevorzugt ist Butylacetat.

Die optional erhöhte Temperatur dient der besseren Löslichkeit des Additivs im Lösemittel und liegt bevorzugt in einem Bereich von 40 bis 100 °C oder bis zur Siedetemperatur des Lösemittels und besonders bevorzugt in einem Bereich von 50 bis 90 °C.

Wird als leafing-Additiv ein organofunktionelles Silan verwendet, so wird dieses bevorzugt durch eine Sol-Gel-Reaktion auf die Oberfläche der geprägten Effektpigmente aufgebracht. Hierbei werden als Lösemittel bevorzugt Ethanol, Isopropanol oder Mischungen hiervon verwendet. Dem Lösungsmittel kann optional geeignete Mengen an Wasser und an saurem und/oder basischem Katalysator zugesetzt werden. Das Wasser bewirkt eine bessere Hydrolyse der Alkoxygruppen des Silans.

Das leafing-Additiv zur Oberflächenmodifizierung wird bevorzugt in einer Menge aus einem Bereich von 5 Gew.-% bis 50 Gew.-%, besonders bevorzugt aus einem Bereich von 10 Gew.-% bis 40 Gew.-% und ganz besonders bevorzugt aus einem Bereich von 15 Gew.-% bis 35 Gew.-%, jeweils bezogen auf das Gesamtgewicht des eingesetzten geprägten Effektpigments, eingesetzt.
Da sich die hier angegebenen Mengen des Additivs auf das Ausgangsmaterial beziehen, kann die tatsächliche Additivmenge des fertig beschichteten geprägten Effektpigments geringer sein, da z.B. bei einer Menge von 50 Gew.-% nicht alles Additiv auf die Pigmentoberfläche aufziehen kann. Dementsprechend können geringere Mengen der leafing-Additive in den die geprägten Effektpigmente enthaltenden erfindungsgemäßen Nagellack gefunden werden.
Die recht hohen Additivmengen im Ausgangsmaterial bewirken jedoch eine sehr hohe und dichte Beschichtung der Effektpigmentoberfläche mit dem Additiv.

Bei einer weiteren besonders bevorzugten erfindungsgemäßen Ausführungsform wird bei dem Beschichtungsverfahren das leafing-Additiv zur Oberflächenmodifizierung von geprägten Effektpigmenten wenigstens ein Phosphonsäureester und/oder Phosphonsäure gemäß der Formel (II) in einer Gesamtmenge aus einem Bereich von 10 Gew.-% bis 50 Gew.-%, besonders bevorzugt aus einem Bereich von 15 Gew.-% bis 45 Gew.-%, und ganz besonders bevorzugt aus einem Bereich von 20 Gew.-% bis 40 Gew.-%, jeweils bezogen auf das Gesamtgewicht des geprägten Effektpigments, eingesetzt.

Unterhalb der angegebenen Mengen an Phosphonsäureester und/oder Phosphonsäure findet kein ausreichendes leafing der Effektpigmente statt. Oberhalb der angegebenen Mengen an Phosphonsäureester und/oder Phosphonsäure können gegebenenfalls zu große Mengen des Phosphonsäureesters und/oder Phosphonsäure in die fertige Nagellackkomposition eingetragen werden.
Hierbei ist es besonders bevorzugt, dass die Effektpigmente in einem separaten Schritt mit dem Phosphonsäureester und/oder Phosphonsäure beschichtet werden, bevor sie in das Nagellacksystem eingetragen werden.

Bei besonders bevorzugten Ausführungsformen werden als leafing-Additiv wenigstens ein Phosphorsäureester der Formel (I), wobei jeweils R ein linearer Alkylrest mit bevorzugt C₁₀ bis C₂₀, besonders bevorzugt C₁₂ bis C₁₈ und R¹ = H ist, in einer Gesamtmenge aus einem Bereich von 15 Gew.-% bis 40 Gew.-%, weiter bevorzugt einem Bereich von 20 Gew.-% bis 35 Gew.-%, bezogen auf das Gesamtgewicht des eingesetzten geprägten Effektpigments, im erfindungsgemäßen Verfahren eingesetzt.

Bei einer weiteren bevorzugten Ausführungsform wird als leafing-Additiv zur Oberflächenmodifizierung der geprägten Effektpigmente basierend auf metallischen plättchenförmigen geprägten Substraten, wenigstens eine Fettsäure in einer Gesamtmenge aus einem Bereich von bevorzugt 5 Gew.-% bis 40 Gew.-%, besonders bevorzugt aus einem Bereich von 10 Gew.-% bis 38 Gew.-% und ganz besonders bevorzugt aus einem Bereich von 20 Gew.-% bis 35 Gew.-%, jeweils bezogen auf das Gesamtgewicht des eingesetzten Effektpigments, eingesetzt.

Bei einer weiteren bevorzugten Ausführungsform wird als leafing-Additiv zur Oberflächenmodifizierung der geprägten Effektpigmente basierend auf metallischen plättchenförmigen geprägten Substraten, wenigstens ein Silan in einer Gesamtmenge bevorzugt aus einem Bereich von 5 Gew.-% bis 39 Gew.-%, weiter bevorzugt aus einem Bereich von 10 Gew.-% bis 35 Gew.-%, besonders bevorzugt aus einem Bereich von 15 Gew.-% bis 32 Gew.-% und ganz besonders bevorzugt aus einem Bereich von 18 Gew.-% bis 30 Gew.-%, jeweils bezogen aus das Gesamtgewicht des eingesetzten geprägten Effektpigments, eingesetzt.

Unterhalb der bei den erfindungsgemäß oberflächenmodifizierten geprägten Effektpigmenten bzw. der verschiedenen, vorstehend beschriebenen Effektpigment-/Additivkombinationen für die jeweils für verschiedene Effektpigmenttypen angegebenen Mengen der als Ausgansmaterial zur Oberflächenmodifizierung zu verwendenden spezifischen Substanzen findet kein ausreichendes leafing der Effektpigmente statt. Oberhalb der jeweils angegebenen Mengen der Additive können gegebenenfalls zu große Mengen der Additive in die fertige Nagellackkomposition eingetragen werden und dort störende Effekte hervorrufen.

Ein Teil der eingesetzten leafing-Additive wird bei den oben angegebenen höheren Eckwerten der Bereiche der Ausgangskonzentrationen nicht auf der Oberfläche des geprägten Effektpigments anhaften, da dessen Oberfläche bereits gesättigt ist. Für eine hohe und gleichmäßige Bedeckung ist es jedoch nötig, ausreichende Mengen an Additiv, die auch einen deutlichen Überschuss beinhalten können, zur Verfügung zu stellen, da nur dann ein starker leafing-Effekt zu erwarten ist.

Naturgemäß kann die Menge des leafing-Additivs verringert werden, je geringer die spezifische Oberfläche des geprägten Effektpigments ist und umgekehrt. Eine geringere spezifische Oberfläche liegt vor bei sehr großen und/oder dicken geprägten Effektpigmente. Dicke geprägte Effektpigmente werden insbesondere bei jenen erhalten, bei denen das metallische geprägte Substrat mit weiteren Schichten beschichtet ist.

Unter "Gesamtmenge" wird erfindungsgemäß die komplette Menge an Ausgangsmaterial des leafing-Additivs verstanden und zwar unabhängig davon, ob es sich ausschließlich um wenigstens einen Phosphorsäureester oder ausschließlich um wenigstens eine Phosphonsäure oder Phosphonsäureester oder um eine Mischung aus wenigstens einem Phosphorsäureester und wenigstens einer Phosphonsäure oder um eine Mischung unterschiedlicher Phosphonsäuren oder um eine Mischung unterschiedlicher Fettsäuren oder um eine Mischung unterschiedlicher Silane handelt.

Die erfindungsgemäß mit dem leafing-Additiv oberflächenmodifizierten geprägten Effektpigmente können neben dem erfindungsgemäßen Nagellack auch in anderen in kosmetischen Formulierungen, Verwendung finden. Die oberflächenmodifizierten Effektpigmente zeichnen sich in Nagellackkompositionen und insbesondere in der erfindungsgemäßen Nagellackkomposition durch ihr ausgezeichnetes leafing-Verhalten aus.

### Bindemittel:

Die erfindungsgemäßen Nagellackkompositionen umfassen als Bindemittel wenigstens ein Kohlenwasserstoffharz, wobei das Bindemittel bevorzugt einen Bindemittelfestkörperanteil aus einem Bereich von 25 Gew.-% bis 64 Gew.-%, weiter bevorzugt aus einem Bereich von 25 Gew.-% bis 60 Gew.-%, weiter bevorzugt aus einem Bereich von 28 Gew.-% bis 55 Gew.-%, besonders bevorzugt aus einem Bereich von 29 Gew.-% bis 50 Gew.-% und ganz besonders bevorzugt aus einem Bereich von 35 Gew.-% bis 43 Gew.-%, jeweils bezogen auf das Gesamtgewicht der Nagellackkomposition, aufweist.
Unterhalb eines Bindemittelanteils von 25 Gew.-% waren keine guten optischen Effekte durch die Effektpigmente im applizierten Nagellack mehr zu erkennen.
Oberhalb von 63 Gew.-% lässt die optische Qualität der Effektpigmente ebenfalls nach und zunehmend wird die Viskosität der erfindungsgemäßen Nagellackkompositionen zu hoch.

Unter Kohlenwasserstoffharze versteht man synthetische Harze, die durch Reaktion von Kohlenwasserstoffen (außer Olefinen) mit sich selbst in Gegenwart von Aluminiumchlorid oder Schwefelsäure als Katalysator entstehen (siehe https://www.spektrum.de/lexikon/chemie/kohlenwasserstoffharze/4959). Die Kohlenwasserstoffharze werden entsprechend ihres Aufbaus in die drei Gruppen Petroleumharze, Kohlenteerharze und Terpenharze unterteilt. Cumaron-Inden-Harze sind die wichtigste Gruppe der Kohlenteerharze. Zu den Kohlenwasserstoffharze werden auch die Reaktionsprodukte aus Xylol und Formaldehyd, die Xylol-Formaldehyd-Harze gerechnet. In bevorzugten Ausführungsformen werden Nagellackkompositionen enthaltend aromatische Kohlenwasserstoffharze verwendet.

Weiterhin besonders bevorzugte aromatische Kohlenwasserstoffharze sind Harze, die überwiegend bis gänzlich durch Polymerisation verschiedener gereinigter Styrolmonomere gewonnen werden. Bevorzugt sind dabei die Styrolmonomeren optisch weitgehend transparent ("wasserklar").

Die Kohlenwasserstoffharze stellen eine bestimmte Gruppe von Harzen dar, die für Lacke und Druckfarben verwendet werden.
Keinesfalls handelt es sich bei dieser speziellen Harzklasse ganz allgemein um Harze, die lediglich auf Kohlenwasserstoffbasis aufgebaut sind.

Die Herstellung der Kohlenwasserstoffharze erfolgt in bekannter Weise durch Erhitzen von hochsiedenden Fraktionen der Benzinpyrolyse (Pyrolyseöl) oder der isoprenfreien C₅-Fraktion der Benzinpyrolyse in Anwesenheit von Aluminiumchlorid. Die Kohlenwasserstoffharze sind in den meisten organischen Lösungsmitteln, z. B. Ester, Ether, Chlorkohlenwasserstoffe und Aromaten, löslich.

Ohne an eine Theorie gebunden zu sein, vermuten die Erfinder, dass bei der Verwendung von polaren Bindemitteln die mit geeigneten Additiven beschichteten Effektpigmente immer noch teilweise von dem Bindemittel benetzt werden und daher nicht den gewünschten leafing- Effekt aufweisen. Hingegen sind die Harze der erfindungsgemäßen Nagellackkomposition ungewöhnlich unpolar für Nagellackkompositionen und benetzen demzufolge die Effektpigmente nicht. Dadurch können vermutlich die geprägten Effektpigmente den leafing-Effekt besser ausbilden.

Gewöhnlicherweise werden als "Kohlenwasserstoffharze" sehr niedrigmolekulare Polymere mit Molmassen unter 2.000 g/mol verstanden. Dagegen hat sich überraschenderweise gezeigt, dass erfindungsgemäß auch Kohlenwasserstoffharze mit höhermolekularen Molmassen verwendet werden können.

Die erfindungsgemäßen Nagellackkompositionen umfassen vorzugsweise als Bindemittel Kohlenwasserstoffharze mit einem mittleren Molekulargewicht (M_{w}) aus einem Bereich von 800 bis 6.500 g/mol, bevorzugt aus einem Bereich von 900 bis 6.000 g/mol oder aus einem Bereich von 1.200 bis 5.500 g/mol. Das mittlere Molekulargewicht Mw wurde mittels Gel-Permeations-Chromatographie (GPC) mit einem Polystyrol-Standard bestimmt.

Bei einer bevorzugten Ausführungsform umfassen die erfindungsgemäßen Nagellackkompositionen wenigstens zwei voneinander verschiedene Kohlenwasserstoffharze mit einem ersten mittleren Molekulargewicht Mw aus einem Bereich von 1.000 bis 2.000 g/mol und einem zweiten mittleren Molekulargewicht Mw aus einem Bereich von 4.000 bis 5.900 g/mol im Gewichtsverhältnis 1:1 bis 1:10, bevorzugt 1:1 bis 1:8, besonders bevorzugt 1:1 bis 1:4 und ganz besonders bevorzugt 1:1 bis 1:2 der beiden verschiedenen Kohlenwasserstoffharze.

Bei einer besonders bevorzugten Ausführungsform umfassen die erfindungsgemäßen Nagellackkompositionen wenigstens zwei voneinander verschiedene Kohlenwasserstoffharze mit einem ersten mittleren Molekulargewicht Mw aus einem Bereich von 1.200 bis 1.600 g/mol und einem zweiten mittleren Molekulargewicht Mw aus einem Bereich von 4.500 bis 5.500 g/mol im Gewichtsverhältnis 1:1 bis 1:10, bevorzugt 1:1 bis 1:8, besonders bevorzugt 1:1 bis 1:4 und ganz besonders bevorzugt 1:1 bis 1:2 der beiden verschiedenen Kohlenwasserstoffharze.
Bevorzugt beziehen sich diese Mischungen aromatische Kohlenwasserstoffharze, die überwiegend bis gänzlich durch Polymerisation verschiedener gereinigter Styrolmonomere gewonnen werden.

Die erfindungsgemäßen Nagellackkompositionen können als Bindemittel Kohlenwasserstoffharze, wie beispielsweise Kristalex F100 Hydrocarbon Resin, Kristalex 5140 Hydrocarbon Resin, Kristalex 3070 Hydrocarbon Resin, Kristalex 3085 Hydrocarbon Resin, Kristalex F115 Hydrocarbon Resin, jeweils Fa. Eastman umfassen. Hierbei handelt es sich um Harze, die überwiegend bis gänzlich durch Polymerisation verschiedener gereinigter Styrolmonomere gewonnen werden.

Bevorzugt umfassen die erfindungsgemäßen Nagellackkompositionen als Bindemittel die Kohlenwasserstoffharze Kristalex F100 Hydrocarbon Resin und Kristalex 5140 Hydrocarbon Resin.

In besonders bevorzugten Ausführungsformen enthält die erfindungsgemäße Nagellackkomposition kohlenwasserstoffhaltiges Harz in einer Menge, die 80 bis 100 Gew.-%, weiter bevorzugt 90 bis 100 Gew.-% und besonders bevorzugt 95 bis 100 Gew.-% des gesamten organischen Bindemittels ausmacht.

Die Verwendung von Kohlenwasserstoffharzen in Nagellacken als Hauptbestandteil des Bindemittels ist nach den Kenntnissen der Erfinder nicht üblich. Gewöhnlicherweise sind Kohlenwasserstoffharze sehr selten Bestandteile von Nagellacken und wenn, so werden sie zusammen mit anderen Bindemitteln in vergleichsweise eher geringen Anteilen eingesetzt.

In weitere Ausführungsformen enthält daher die erfindungsgemäße Nagellackkomposition keine oder nahezu keine zusätzlichen Bindemittel der Gruppe aus Nitrocellulose, Polyesterharze, Polyvinylharze, Alkydharz, Epoxidharze oder Celluloseacetatbutyrat. Diese Bindemittel sind bevorzugt in Mengenanteilen von unter 10 Gew.-%, weiter bevorzugt unter 5 Gew.-% und besonders bevorzugt von unter 1 Gew.-% und ganz besonders bevorzugt unter 0,1 Gew.-%, jeweils bezogen auf das Gesamtgewicht der Kohlenwasserstoffharze sowie zusätzliche Bindemittel, enthalten. Diese Bindemittel haben sich eher als hinderlich zur Erzielung eines wirklich starken Regenbogeneffekts erwiesen.

Ohne an eine Theorie gebunden zu sein vermuten die Erfinder, dass bei Nagellackkompositionen die die oben genannten Bindemittel enthalten, diese aufgrund ihrer stärkeren Polarität zumindest teilweise die Effektpigmente benetzen und diese dadurch schlechtere leafing-Eigenschaften aufweisen.

### Lösemittel:

Die erfindungsgemäßen Nagellackkompositionen enthalten bevorzugt bestimmte Lösemittel. Als Lösungsmittel können den erfindungsgemäßen Nagellackkompositionen beispielsweise Ethylacetat, Butylacetat oder Isopropanol zugesetzt werden.

Bevorzugt enthält die erfindungsgemäße Nagellackkomposition als Lösemittel eine Mischung aus Isopropanol, Ethylacetat und Butylacetat.

Besonders bevorzugt enthält die erfindungsgemäße Nagellackkomposition die Lösemittelmischung aus Isopropanol, Ethylacetat und Butylacetat in einer Menge, die 70 bis 100 Gew.-%, weiter bevorzugt 75 bis 98 Gew.-%, bezogen auf das gesamte Lösemittel der Nagellackkomposition, beträgt.

Hierbei ist es unerheblich, ob diese bevorzugten Lösemittel über die Bindemittel oder die Effektpigmentdispersion eingetragen werden.

In weiteren bevorzugten Ausführungsformen beträgt bei dieser Lösemittelmischung der Anteil an Butylacetat 30 bis 60 Gew.-% und besonders bevorzugt 35 bis 55 Gew.-%, bezogen auf das gesamte Lösemittel.

Bei einer weiteren besonders bevorzugten Ausführungsform liegt der Anteil von Isopropanol unter 20 Gew.-%, vorzugsweise unter 15 Gew.-%, und weiter bevorzugt unter 10 Gew.-%, jeweils bezogen auf das gesamte Lösemittel.
Zu hohe Anteile an Isopropanol in der erfindungsgemäßen Nagellackkomposition führen zu einem schlechten optischen Erscheinungsbild der Effektpigmente. Vermutlich ist dies auf eine zu schnelle Trocknung der Nagellacke nach ihrer Applikation zurückzuführen.

Die erfindungsgemäßen Nagellackkompositionen sind äußerst einfach auf einem menschlichen oder künstlichen Finger- und/oder Fußnagel applizierbar. Sie zeichnen sich während der Applikation durch einen guten Verlauf aus und bilden nach der anschließenden Trocknung einen homogenen Film auf einem menschlichen oder künstlichen Finger- und/oder Fußnagel.

In bevorzugten Ausführungsformen enthält der erfindungsgemäße Nagellack 50 Gew.-% bis 70 Gew.-%, bevorzugt 55 Gew.-% bis 68 Gew.% und besonders bevorzugt 57 bis 65 Gew.-% Lösemittel, jeweils bezogen auf das Gewicht des gesamten Nagellacks.

Unterhalb eines Lösemittelgehalts von 55 Gew.-% steigt die Viskosität des Nagellacks zu stark an und die Effektpigmente können sich nicht optimal orientieren, was zu einer Verminderung bis Verlust des Regenbogeneffekts führt.

Oberhalb eines Lösemittelgehalts von 70 Gew.-% verringert sich die Viskosität des Nagellacks zu sehr, was zu einem schlecht kontrollierbaren Auftrag des Nagellacks auf den Fingernagel führt.

### Weitere Bestandteile:

Die erfindungsgemäßen Nagellackkompositionen können zusätzlich einen oder mehrere weitere Bestandteile enthalten. Hier sind insbesondere Weichmacher und Antioxidantien zu nennen.

Als Weichmacher können beispielsweise Glycole und ihre Derivate wie beispielsweise Diethylenglycolethylether, Diethylenglycolmethylether, Diethyleneglycolbutylether oder weiterhin Diethylenglycolhexylether, Ethylenglycolethylether, Ethylenglycolmethylether, Ethylenglycolbutylether, Ethylenglycolhexylether, Glycolesters, Derivate von Propylenglycol und insbesondere Propylenglycolphenylether, Propylenglycoldiacetate, Dipropylenglycolbutylether, Tripropylenglycolbutylether, Propylenglycolmethylether, Dipropylenglycolethylether, Tripropylenglycolmethylether und Diethylenglycolmethylether, Propylenglycobutylether, oder Mischungen hiervon, verwendet werden. Weiterhin können als Weichmacher insbesondere Ester von Carbonsäuren, wie beispielsweise von Citraten, insbesondere von Trimethylcitrat, Tributylcitrat, Trimethylacetylcitrat, Tributylacetylcitrat, Triethyl-2-hexylacetylcitrate oder von Phthalaten, insbesondere Dimethoxyethylphthalat; oder von Phosphaten, insbesonmdere von Tricresylphosphat, Tributylphosphat, Triphenylphosphat, Tributoxyethylphosphate oder von Tartraten, insbesondere Dibutoxytartrat; Adipaten, Carbonaten, Sebacaten; Benzylbenzoat, Butylacetylricinoleat, Glycerylacetylricinoleat, Butylglycolat, Kampher, Glyceroltriacetate, N-ethyl-o,p-toluolsulfonamid, Oxyethylen Verbindungen wie beispielsweise Oxyethylenöle, insbesondere pflanzliche Öle, wie beispielsweise Castoröl, Kohlenwasserstofföle und Mischungen hiervon.
Bevorzugte Weichmacher sind insbesondere Kohlenwasserstofföle.
Die Gewichtsanteile der Weichmacher an der gesamten Nagellackkomposition betragen bevorzugt einen Bereich von 0 bis 15 Gew.-%, weiter bevorzugt von 1 bis 10 Gew.-%, und besonders bevorzugt von 5 bis 10 Gew.-%.

Die erfindungsgemäße Nagellackkomposition kann weiterhin ein oder mehrere Antioxidantien enthalten.
Unter "Antioxidantien" werden Verbindungen verstanden, die die Bestandteile des erfindungsgemäßen Nagellacks, insbesondere die Kohlenwasserstoffbindemittel, vor dem Einfluss von Sauerstoff, Hitze, Ozon, und/oder UV-Strahlung schützen. Es können eine oder mehrere derartige Verbindungen verwendet werden.
Beispiele für derartige Verbindungen sind IRGANOX® 1010, IRGANOX® 565, IRGANOX® 1076 (Fa. BASF) oder Schwefel-haltige Antioxidantien wie beispielsweise Zinkdibutyldithiocarbamat (PERKACIT ZDBC.(Fa. Performance additives Italy S.p.A). Bevorzugt werden die Antioxidantien in Mengen aus einem Bereich von 0 bis 5 Gew-%, weiter bevorzugt aus einem Bereich von 0,05 bis 1 Gew.-%, bezogen auf die gesamte Nagellackkomposition, eingesetzt.

### Weitere Additive:

Die erfindungsgemäße Nagellackkomposition kann weiterhin übliche weitere Additive enthalten, wie sie dem Fachmann bekannt sind.
Derartige weitere Additive sind beispielsweise Antiabsetzmittel, Konservierungsstoffe, Öle, Wachse, Radikalfänger, Benetzungsadditive, Dispergierhilfsmittel, Benetzungshilfsmittel, Antischaummittel, Parfüm, Neutralisiermittel, Verdicker, UV-Blocker Feuchthaltemittel, Vitamine, Proteine und Mischungen hiervon.

Im Unterschied zu den leafing-Additiven werden diese weiteren Additive der Nagellackkomposition erst während der Formulierung dieser Komposition zugesetzt und nicht separat vorab dem geprägten Effektpigment.

In weiteren Ausführungsformen enthält die erfindungsgemäße Nagellackkomposition vorzugsweise keine Antiabsetzmittel. Etwaig abgesetztes oberflächenmodifiziertes Effektpigmente lassen sich erstaunlicherweise in aller Regel auch ohne den Zusatz von Antiabsetzmitteln durch einfaches Aufschütteln wieder dispergieren.

Die erfindungsgemäße Nagellackkomposition weist vorzugsweise eine Viskosität von 10 sec bis 16 sec auf, gemessen mit einem DIN Auslaufbecher (DIN 4 mm) gemäß DIN 53211.

In bevorzugten Ausführungsformen enthält die erfindungsgemäße Nagellackkomposition geprägte Aluminium-PVD Effektpigmente mit einem periodischen Muster mit diffraktiven Elementen, die bevorzugt Linien mit einer Liniendichte von 11.000 bis 16.000 Linien/cm sind. Diese geprägten Aluminium-PVD Effektpigmente werden bevorzugt mit Laurylphosphonsäuremethylester, Laurylphosphonsäureethylester Laurylphosphonsäure (R = 12) oder Monocetylphosphorsäureester, Dicetylphosphorsäureester (R = 16) und Mischungen hiervon als leafing-Additiv beschichtet. Als Bindemittel werden aromatische Kohlenwasserstoffharze auf Basis von Styrolmonomeren und als Lösemittel eine Mischung aus Isopropanol, Ethylacetat und Butylacetat verwendet, wobei diese Lösemittelmischung 70 bis 100 Gew.-% des gesamten Lösemittels der Nagellackkomposition ausmacht.

Bei einer weiteren bevorzugten Ausführungsform enthält die erfindungsgemäße Nagellackkomposition Aluminium-PVD Effektpigmente mit einer mittleren Dicke h₅₀ aus einem Bereich von 14 bis 40 nm, bevorzugt aus einem Bereich von 15 bis 35 nm, sowie wenigstens zwei voneinander verschiedene aromatische Kohlenwasserstoffharze und eine Mischung aus Isopropanol, Ethylacetat und Butylacetat als Lösemittel, wobei diese Lösemittelmischung 70 bis 100 Gew.-% des gesamten Lösemittels der Nagellackkomposition ausmachen. Bevorzugt werden auch hier als leafing-Additiv Phosphorsäurecetylester oder Laurylphosphonsäure verwendet.

### Verfahren:

Gegenstand der Erfindung ist weiterhin ein Verfahren zur Herstellung der erfindungsgemäßen Nagellackkomposition, umfassend die Schritte
i) Oberflächenmodifizierung des geprägten Effektpigments durch ein Additiv in einer Dispersion in einem Lösemittel,
ii) Lösen des Kohlenwasserstoffharzes in einem Lösemittel oder Lösemittelgemisch
iii) Vermischen und Homogenisieren der Dispersion nach i) mit der Bindemittellösung nach ii).

Das Lösen des Kohlenwasserstoffharzes in einem Lösemittel nach Schritt ii) erfolgt bevorzugt in einem (Zahl: 1) Lösemittel oder einem Gemisch aus mindestens zwei, bevorzugt drei Lösemitteln. Besonders bevorzugt wird als Lösemittelmischung eine Mischung aus Isopropanol, Ethylacetat und Butylacetat verwendet.

Als einzelnes Lösemittel wird bevorzugt Butylacetat verwendet.

In weiteren bevorzugten Ausführungsformen wird das Lösemittel des Schrittes i) ebenfalls aus Isopropanol, Ethylacetat und Butylacetat oder einer Mischung hiervon bestehen, um nicht weitere, möglicherweise störende Lösemittel in den Nagellack einzutragen.

Bevorzugt wird hierbei der Schritt i) gemäß folgendem, weiter oben bereits besprochenen Verfahren ausgeführt.

Ein erfindungsgemäßes Verfahren zur Oberflächenmodifizierung der geprägten Effektpigmente umfasst die folgenden Schritte:
i. Suspendieren des geprägten Effektpigments umfassend ein plättchenförmiges metallisches Substrat mit geprägter Struktur in wenigstens einem Lösemittel,
ii. Zugabe des leafing-Additivs bei optional erhöhter Temperatur zu der Suspension aus Schritt i. und Rühren der nun erhaltenen Suspension,
iii. Abtrennen und optional Trocknen, des gemäß Schritt ii. erhaltenen oberflächenmodifizierten geprägten Effektpigments vom Lösemittel.

Weiterhin ist es bevorzugt, bei der Auswahl der metallischen geprägten Effektpigmente solche zu wählen, die in ihrer ursprünglichen Dispersion in einem Lösemittel gemäß der bevorzugten Gruppe Isopropanol, Ethylacetat und Butylacetat oder Mischungen davon vorliegen, da das Lösemittel der Metalleffektpigmentdispersion ebenfalls in geringen Mengen in den erfindungsgemäßen Nagellack gelangt, falls nicht aufwendige Umnetzungsschritte eingesetzt werden.

Insbesondere beim Einsatz von einschichtigen geprägten Metalleffektpigmenten, insbesondere einschichtigen geprägten Aluminiumeffektpigmenten liegen diese durch PVD-Verfahren hergestellten Effektpigmente stets in einer Dispersion vor, da sie als Pulver zur Agglomeration neigen.

Beschrieben wird ebenfalls ein Verfahren zur Beschichtung eines natürlichen oder künstlichen Fingernagels umfassend die Schritte:
a) Beschichtung des natürlichen oder künstlichen Fingernagels mit einer erfindungsgemäßen Nagellackkomposition und anschließendem Trocknen des Nagellacks,
b) optional darauffolgende Beschichtung des Nagellacks mit einem Klarlack.

Das Aufbringen des Klarlacks erhöht beträchtlich die Abriebbeständigkeit der Nagellackierung. Aufgrund des ausgeprägten leafing-Effektes der Effektpigmente im Nagellack weist dieser naturgemäß eine eher geringe Abriebbeständigkeit auf.

Vor dem Schritt a) kann ebenfalls schon eine Beschichtung des natürlichen oder künstlichen Fingernagels mit einem Klarlack erfolgen, um eine möglichst ebene Oberfläche herzustellen. Diese Vorgehensweise empfiehlt sich, falls die Fingernägel eine hohe Rauigkeit aufweisen.

Die nachfolgende Beschichtung mit Klarlack in Schritt b) kann mit dem gleichen oder einem anderen Klarlack wie der erfindungsgemäße Nagellack durchgeführt werden. Allerdings enthält dieser Klarlack auf keinen Fall Effektpigmente, da diese den gewünschten Effekt des erfindungsgemäßen Nagellacks überlagern würden.

Allerdings kann der Klarlack in Schritt b) konventionelle Farbpigmente oder Farbstoffe enthalten. Gerade in Kombination mit metallischen PVD-Aluminiumpigmenten oder mit dünnen, durch Nassvermahlung hergestellten Aluminiumeffektpigmenten mit einem h₅₀-Wert von 20 bis unter 100 nm lassen sich optisch sehr reizvolle Effekte realisieren. Bevorzugt weisen hierbei die mit den Effektpigmenten pigmentierte erfindungsgemäße Nagellackkompositionen nach Schritt a) einen Spiegelglanz auf.

Bei einer weiteren Ausführungsform kann die erfindungsgemäße Nagellackkomposition mit einem niedrig viskosen UV härtenden Klarlack überlackiert werden um die Abriebbeständigkeit der erfindungsgemäßen Nagellackkomposition zu erhöhen.

Bevorzugt können auch lösemittelbasierende Klarlacke verwendet werden. Ohne an eine Theorie gebunden zu sein, basiert der Klarlack bevorzugt auf polaren Bindemitteln, die nur gering mit den Kohlenwasserstoffharzen des erfindungsgemäßen Klarlacks wechselwirken. In besonders bevorzugten Ausführungsformen basierend diese Klarlacke auf Bindemitteln wie Polyvinyl Butyral (PVB), Polyvinylpyrrolidon (PVP) oder Mischungen hiervon.

Weiterhin enthält der Klarlack bevorzugt Lösemittel, die nicht die unpolaren Kohlenwasserstoffharze des erfindungsgemäßen Lacks lösen bzw. anlösen. Beispielsweise kann hierfür bevorzugt Isopropanol verwendet werden. Andernfalls können auch die leafing-Effektpigmente wieder angelöst und in ihrer Orientierung gestört werden, wodurch der Spiegelglanzeffekt gestört wird.

Überraschenderweise weisen diese bevorzugten Klarlacke eine sehr gute Haftungsbeständigkeit auf dem erfindungsgemäßen Nagellack auf.

### Bevorzugte geprägte oberflächenmodifizierte Effektpigmente:

Im Rahmen der Aufgabe der vorliegenden Erfindung liegt es, ein geprägtes Effektpigment für die Verwendung in einer Nagellackkomposition bereitzustellen, welches einen verbesserten Regenbogeneffekt sowie ein verbessertes Chroma und eine verbesserte Brillanz aufweist.

Ein bevorzugtes geprägtes Effektpigment umfasst ein plättchenförmiges metallisches Substrat mit geprägter Struktur, welches durch PVD-Verfahren hergestellt wird und mittels Beschichtung mit einem leafing-Additiv aus Phosphorsäureestern der allgemeinen Formel:

(R-O)ₓ-P(O)(OR¹)₍₃₋ₓ₎ (I)

wobei x = 1 oder 2 ist, R = linearer und/oder verzweigter Alkylrest mit einer Kohlenstoffkette aus einem Bereich von C₁₂ bis C₂₀ und R¹ = H ist.

Bevorzugt handelt es sich bei diesem geprägten Effektpigment um ein einschichtiges Effektpigment mit einem metallischen Substrat aus Aluminium. Weiterbevorzugt sind die geprägte Struktur Linien, die eine Liniendichte von 5.000 bis 20.000 Linien/cm aufweisen. Die Linien sind bevorzugt Wellenlinien ("sinusförmig"), da diese Struktur besonders einfach herzustellen ist.

Erfindungsgemäß wird dieses bevorzugte geprägte Effektpigment mit einem leafing-Additiv zur Oberflächenmodifizierung behandelt, welches aus Monocetylphosphorsäureester, Dicetylphosphorsäureester (R = 16) und Mischungen hiervon, besteht.

In einer weiter bevorzugten Ausführungsform wird das geprägte oberflächenmodifizierte Effektpigment hergestellt in einem Verfahren welches die folgenden Schritte umfasst:
i. Suspendieren des geprägten Effektpigments umfassend ein plättchenförmiges metallisches Substrat mit geprägter Struktur in wenigstens einem Lösemittel,
ii. Zugabe des leafing-Additivs bei optional erhöhter Temperatur zu der Suspension aus Schritt i. und Rühren der nun erhaltenen Suspension,
iii. Abtrennen und optional Trocknen, des gemäß Schritt ii. erhaltenen oberflächenmodifizierten geprägten Effektpigments vom Lösemittel.

Hierbei wird das leafing-Additiv, bezogen auf die Gesamtmenge an Substrat in einem Bereich von 15 Gew.-% bis 50 Gew.-% eingesetzt werden.

Ein Überschuß an Additiv ist nötig, um einen stabilen leafing-Effekt zu bewirken.

### Beispiele:

Die nachfolgenden Beispiele dienen der näheren Beschreibung der Erfindung und sollen in keinerlei Hinsicht einschränkend sein. Alle Prozentangaben sind Gewichtsprozentangaben. Die Begriffe NFA (nichtflüchtiger Anteil), Festkörperanteil und Feststoffgehalt sind austauschbar verwendbar.

### I Herstellung der oberflächenmodifizierten Effektpigmente

### Beispiel 1:

In einem 1L-Doppelwandreaktor wurden 198 g der PVD- Aluminiumeffektpigment-dispersion kommerziell erhältliches Metalure Prismatic H- 50550 AE (Dispersion in Essigsäureethylester, Feststoffgehalt 5,05 Gew.-%, D₅₀ (Horiba LA-950) = ca. 50 µm, Fa. ECKART America) in einem Lösemittel gemäß nachstehender Tabelle 1 bei 200 rpm/min dispergiert und auf 80°C erhitzt. Anschließend wurde das Additiv Phosphorsäurecetylester (CAS Nummer: 3539-43-3, Hostaphat CC 100, Fa. Clariant) gemäß nachstehender Tabelle 1, gelöst in 20 g des zum Dispergieren verwendeten Lösemittels (AE), zur Aluminiumeffektpigment-Dispersion zugegeben. Nach 6 Stunden rühren bei 80°C wurde die Mischung abgekühlt und über einen Büchnertrichter filtriert. Es wurden oberflächenmodifizierte PVD-Aluminiumeffektpigmente als 5-25%ige Dispersionen erhalten. Diese wurde mit Ethylacetat zu einer Pigmentdispersion mit einem NFA von 5% ergänzt.

### Beispiele 2 bis 10:

Es wurde wie in Beispiel 1 verfahren, jedoch wurde teilweise als PVD-Aluminiumeffektpigment kommerziell erhältliches Silverdream Prismatic H-50720 (Dispersion in Essigsäureethylester, Feststoffgehalt 7,05 Gew.-%, D₅₀ (Horiba LA-950) = ca. 20 µm, Fa. ECKART America) verwendet und als Additiv teilweise Laurylphosphonsäure (abgekürzt: LPS) sowie als Lösemittel Butylacetat (BA) anstelle von Ethylacetat (AE) verwendet. Die Details zu den verwendeten Substanzen, deren Mengen sowie der Adsorptionstemperatur finden sich in nachfolgender Tabelle 1.

Die beim abschließenden Ergänzen des abgenutschten Produkts zur Pigmentdispersion verwendeten Lösemittel sowie die sich einstellenden nichtflüchtigen Anteile (NFA) der Dispersionen finden sich jeweils in den Spalten 4 und 3 der Tabelle 2 und der Tabelle 3.

Vergleichsbeispiel 1: Hier wurde die unbeschichtete kommerziell erhältliche PVD-Aluminiumeffektpigmentdispersion Metalure Prismatic H- 50550 AE mit eingraviertem Wellenliniengitter (Liniendichte: 12.500 Linien/cm) verwendet.

Vergleichsbeispiel 2: Hier wurde die unbeschichtete kommerziell erhältliche PVD-Aluminiumeffektpigmentdispersion Silverdream Prismatic H-50720 mit eingraviertem Wellenliniengitter (Liniendichte: 12.500 Linien/cm) verwendet.

**Tabelle 1: Versuchsparameter zur Beschichtung der eingravierten PVD-Aluminiumpigmente mit Additiv**

| **Beispiel** | **Verwendetes graviertes PVD-Pigment** | **Menge PVD-Pigment-dispersion / Menge Al** | **Menge Lösemittel zum Dispergieren des PVD-Pigments [g]** | **Additiv** | **Menge Additiv [in Gew.-%, bez. auf Aluminium]** | **Lösemittel zum Lösen Additiv** | **Temperatur Beschichtung [°C]** |
|---|---|---|---|---|---|---|---|
| 1 | Metalure Prismatic H-50550 AE | 198g/10g | 269,3 | Hostaphat CC 100 | 20% | AE* | 60 |
| 2 | Metalure Prismatic H-50550 AE | 198 g/10g | 269,3 | Hostaphat CC 100 | 30% | AE | 60 |
| 3 | Silverdream Prismatic H-50720 | 141,8/10g | 325,5 | Hostaphat CC 100 | 20% | BA | 80 |
| 4 | Silverdream Prismatic H-50720 | 141,8/10g | 325,5 | Hostaphat CC 100 | 30% | BA** | 80 |
| 5 | Silverdream Prismatic H-50720 | 141,8/10g | 325,5 | LPS | 20% | BA | 80 |
| 6 | Silverdream Prismatic H-50720 | 141,8/10g | 325,5 | LPS | 30% | BA | 80 |
| 7 | Metalure Prismatic H-50550 AE | 198g/10g | 269,3 | Hostaphat CC 100 | 20% | BA | 80 |
| 8 | Metalure Prismatic H-50550 AE | 198g/10g | 269,3 | Hostaphat CC 100 | 30% | BA | 80 |
| 9 | Metalure Prismatic H-50550 AE | 198g/10g | 269,3 | LPS | 20% | BA | 80 |
| 10 | Metalure Prismatic H-50550 AE | 198g/10g | 269,3 | LPS | 30% | BA | 80 |

| | | | | | | | |
|---|---|---|---|---|---|---|---|
| *AE: Ethylacetat; **BA: Butylacetat 85/15: Mischung aus 85% Butylacetat und 15% n-Butanol | | | | | | | |

### II Herstellung der erfindungsgemäßen Nagellackkompositionen

### IIa Herstellung des erfindungsgemäßen Nagelklarlacks:

In einem geeigneten Rührgefäß wurde eine 70 Gew.-%-ige Bindemittellösung BM1 hergestellt. Hierzu wurden 30 g Butylacetat 85/15 vorgelegt, unter Rühren und Kühlen (12 °C) mit dem Dissolver Dispermat CNf2 (Fa. Getzmann GmbH) 70 g des Bindemittels Kristalex F100 Hydrocarbon Resin (M_{w} = ca 1.300 g/mol, Fa. Eastman) zugegeben und anschließend 30 Minuten bei 3000 bis 4000 rpm/min nachgerührt.
In einem zweiten geeigneten Rührgefäß wurde eine 60 Gew.-%-ige Bindemittellösung **BM2** hergestellt, Hierzu wurden 40 g Butylacetat 85/15 vorgelegt, unter Rühren und Kühlen (12 °C) mit dem Dissolver Dispermat CNf2 (Fa. Getzmann GmbH) 60 g des Bindemittels Kristalex 5140 Hydrocarbon Resin (M_{w} = ca 4.900 g/mol, Fa. Eastman) zugegeben und anschließend 30 Minuten bei 3000 bis 4000 rpm/min nachgerührt. Der nichtflüchtige Anteil (Bindemittelfestkörperanteil) der vorstehend beschriebenen Bindemittellösungen wurde gemäß DIN EN ISO 3251:2008 bestimmt.

Vergleichsnagelklarlack: Als ein Vergleichslack wurde der unpigmentierte Nagelklarlack Nail Polish Base 18840 der Firma International Lacquers verwendet. Dieser Nagellack enthält als Bindemittel Nitrocellulose und die Lösemittel Ethylacetat, n-Butylacetat sowie Propan-2-ol.

### IIb Herstellung der erfindungsgemäßen pigmentierten Nagellackkompositionen

Zur Herstellung der erfindungsgemäßen Nagellackkompositionen gemäß Beispiel 11 wurden 5,49 g des **BM1** sowie12,84 g des **BM2** vorgelegt und alles durch Rühren gut dispergiert. Anschließend wurden 2,08 g des oberflächenmodifizierten PVD-Pigments aus Beispiel 1 und nachfolgend 2,04 g Isopropylalkohol, 3,27 g N-Butylacetat 85/15 und 0,87 g Ethylacetat zugegeben und der Nagellack gut dispergiert.

Die Nagellackkompositionen gemäß der Beispiele 11 bis 20 wurden analog zu Beispiel 11 hergestellt, wobei die Mengen der einzelnen Komponenten so gewählt wurden, dass sich die gemäß nachstehender Tabelle 2 aufgeführten Mengenverhältnisse jeweils in Gew.-%, bezogen auf die gesamte Nagellackkomposition, ergaben.

Als Vergleichsbeispiele 3 bis 12 wurden ebenfalls definierte Mengen des jeweiligen oberflächenmodifizierten geprägten PVD-Aluminiumeffektpigments (gemäß der Beispiele 1 bis 10) sowie nicht-oberflächenmodifizierter geprägter PVD-Aluminiumeffektpigmente (Vergleichsbeispiele 1 und 2) gemäß nachstehender Tabelle 3 vorgelegt und in den Vergleichsnagellack eingearbeitet.

Als weitere Vergleichsbeispiele wurden die nicht mit Additiven oberflächenmodifizierten Effektpigmente der Vergleichsbeispiele 1 und 2 jeweils mit dem erfindungsgemäßen Nagellack (Vergleichsbeispiele 14 und 15) sowie dem Vergleichsnagelklarlack (Vergleichsbeispiel 16 und 17) eingearbeitet.

Die genauen Mengenangaben (in Gew.-%) finden sich in den nachfolgenden Tabellen 2 und 3. (Das Vergleichsbeispiel 13 ist nicht vorhanden).

**Tabelle 2: Mengenverhältnisse in Gew.-% der zugegebenen Komponenten der erfindungsgemäßen Nagellacke**

| **Probe** | **Verwendetes PVD-Pigment gemäß** | **NFA PVD-Pigment-dispersion %** | **LSM***** | **Einwaage PVD-Pigment-dispersion%** | **Zugabe AE* %** | **Zugabe BA** 85/15 %** | **Zugabe Iso-propanol %** | **BM1 %** | **BM2 %** | **Σ%** |
|---|---|---|---|---|---|---|---|---|---|---|
| Vergleichsbeispiel 14 | Vergleichsbeispiel 1 | 5 | AE | 11,52 | | 12,88 | 3,39 | 21,64 | 50,59 | 100,0 |
| Vergleichsbeispiel 15 | Vergleichsbeispiel 2 | 7 | AE | 8,23 | 3,31 | 12,88 | 3,39 | 21,64 | 50,59 | 100,0 |
| Beispiel 11 | Beispiel 1 | 7,13 | AE | 8,08 | 3,43 | 12,88 | 3,39 | 21,64 | 50,59 | 100,0 |
| Beispiel 12 | Beispiel 2 | 7,94 | AE | 7,25 | 4,25 | 12,88 | 3,39 | 21,64 | 50,59 | 100,0 |
| Beispiel 13 | Beispiel 3 | 6,41 | BA 85/15 | 8,98 | 10,99 | 4,49 | 3,39 | 21,64 | 50,59 | 100,0 |
| Beispiel 14 | Beispiel 4 | 7 | BA 85/15 | 8,23 | 10,99 | 5,20 | 3,39 | 21,64 | 50,59 | 100,0 |
| Beispiel 15 | Beispiel 5 | 7,35 | BA 85/15 | 7,84 | 10,99 | 5,67 | 3,39 | 21,64 | 50,59 | 100,0 |
| Beispiel 16 | Beispiel 6 | 7,31 | BA 85/15 | 7,88 | 10,99 | 5,55 | 3,39 | 21,64 | 50,59 | 100,0 |
| Beispiel 17 | Beispiel 7 | 6,68 | BA 85/15 | 8,62 | 10,99 | 4,84 | 3,39 | 21,64 | 50,59 | 100,0 |
| Beispiel 18 | Beispiel 8 | 4,27 | BA 85/15 | 13,49 | 10,99 | 0 | 3,39 | 21,64 | 50,59 | 100,0 |
| Beispiel 19 | Beispiel 9 | 6,64 | BA 85/15 | 8,67 | 10,99 | 4,84 | 3,39 | 21,64 | 50,59 | 100,0 |
| Beispiel 20 | Beispiel 10 | 6,25 | BA 85/15 | 9,21 | 10,99 | 4,25 | 3,39 | 21,64 | 50,59 | 100,0 |

| | | | | | | | | | | |
|---|---|---|---|---|---|---|---|---|---|---|
| *AE: Ethylacetat; **BA: Butylacetat; *** LSM: Lösemittel der Pigmentdispersion des beschichteten PVD-Pigments | | | | | | | | | | |

**Tabelle 3: Mengenverhältnisse in Gew.-% der zugegebenen Komponenten für die Vergleichsbeispiele der Nagellacke**

| **Probe** | **Verwendetes PVD-Pigment gemäß** | **NFA %** | **LSM** | **Einwaage PVD-Pigment-dispersion%** | **Zugabe AE %** | **Zugabe BA 85/15 %** | **Zugabe Isoprop. %** | **Base 18840 %** | **Σ%** |
|---|---|---|---|---|---|---|---|---|---|
| Vergleichsbeispiel 16 | Vergleichsbeispiel 1 | 5 | AE | 9,75 | 1,67 | - | - | 88,58 | 100,0 |
| Vergleichsbeispiel 17 | Vergleichsbeispiel 2 | 7 | AE | 6,96 | 4,46 | - | - | 88,58 | 100,0 |
| Vergleichsbeispiel 3 | Beispiel 1 | 7,13 | AE | 6,84 | 4,58 | - | - | 88,58 | 100,0 |
| Vergleichsbeispiel 4 | Beispiel 2 | 7,94 | AE | 6,14 | 5,28 | - | - | 88,58 | 100,0 |
| Vergleichsbeispiel 5 | Beispiel 3 | 6,41 | BA 85/15 | 7,61 | - | 3,81 | - | 88,58 | 100,0 |
| Vergleichsbeispiel 6 | Beispiel 4 | 7 | BA 85/15 | 6,96 | - | 4,46 | - | 88,58 | 100,0 |
| Vergleichsbeispiel 7 | Beispiel 5 | 7,35 | BA 85/15 | 6,63 | - | 4,79 | - | 88,58 | 100,0 |
| Vergleichsbeispiel 8 | Beispiel 6 | 7,31 | BA 85/15 | 6,67 | - | 4,75 | - | 88,58 | 100,0 |
| Vergleichsbeispiel 9 | Beispiel 7 | 6,68 | BA 85/15 | 7,30 | - | 4,12 | - | 88,58 | 100,00 |
| Vergleichsbeispiel 10 | Beispiel 8 | 4,27 | BA 85/15 | 11,42 | - | 0,00 | - | 88,58 | 100,0 |
| Vergleichsbeispiel 11 | Beispiel 9 | 6,64 | BA 85/15 | 7,34 | - | 4,08 | - | 88,58 | 100,0 |
| Vergleichsbeispiel 12 | Beispiel 10 | 6,25 | BA 85/15 | 7,80 | - | 3,62 | - | 88,58 | 100,0 |

**Tabelle 4: Finale Mengenverhältnisse der einzelnen Komponenten der erfindungsgemäßen Nagellackkompositionen und der Vergleichsbeispiele in Gew.-%, bezogen auf die gesamte Nagellackkomposition**

| **Probe** | **Al-Gehalt %** | **BA % Gesamt** | **Kristalex F100 %** | **Kristalex 5140 %** | **Isopropanol % Gesamt** | **AE % Gesamt** | **Base 18840 % Gesamt** | **Σ %** |
|---|---|---|---|---|---|---|---|---|
| Vergleichsbeispiel 14 | 0,58 | 39,61 | 15,15 | 30,36 | 3,39 | 12,12 | -- | 100,0 |
| Vergleichsbeispiel 15 | 0,58 | 39,61 | 15,15 | 30,36 | 3,39 | 12,15 | -- | 100,0 |
| Beispiel 11 | 0,58 | 39,61 | 15,15 | 30,36 | 3,39 | 12,12 | -- | 100,0 |
| Beispiel 12 | 0,58 | 39,61 | 15,15 | 30,36 | 3,39 | 12,13 | -- | 100,0 |
| Beispiel 13 | 0,58 | 39,52 | 15,15 | 30,36 | 3,39 | 10,99 | -- | 100,0 |
| Beispiel 14 | 0,58 | 39,57 | 15,15 | 30,36 | 3,39 | 10,99 | -- | 100,0 |
| Beispiel 15 | 0,58 | 39,57 | 15,15 | 30,36 | 3,39 | 10,99 | -- | 100,0 |
| Beispiel 16 | 0,58 | 39,57 | 15,15 | 30,36 | 3,39 | 10,99 | -- | 100,0 |
| Beispiel 17 | 0,58 | 39,57 | 15,15 | 30,36 | 3,39 | 10,99 | -- | 100,0 |
| Beispiel 18 | 0,58 | 39,57 | 15,15 | 30,36 | 3,39 | 10,99 | -- | 100,0 |
| Beispiel 19 | 0,58 | 39,57 | 15,15 | 30,36 | 3,39 | 10,99 | -- | 100,0 |
| Beispiel 20 | 0,58 | 39,57 | 15,15 | 30,36 | 3,39 | 10,99 | -- | 100,0 |
| Vergleichsbeispiel 16 | 0,49 | 0,00** | -- | -- | -- ** | 10,93** | 88,58* | 100,0 |
| Vergleichsbeispiel 17 | 0,49 | 0,00 | -- | -- | -- | 10,94 | 88,58 | 100,0 |
| Vergleichsbeispiel 3 | 0,49 | 0,00 | -- | -- | -- | 10,93 | 88,58 | 100,0 |
| Vergleichsbeispiel 4 | 0,49 | 0,00 | -- | -- | -- | 10,93 | 88,58 | 100,0 |
| Vergleichsbeispiel 5 | 0,49 | 10,93 | -- | -- | -- | 0,00 | 88,58 | 100,0 |
| Vergleichsbeispiel 6 | 0,49 | 10,94 | -- | -- | -- | 0,00 | 88,58 | 100,0 |
| Vergleichsbeispiel 7 | 0,49 | 10,94 | -- | -- | -- | 0,00 | 88,58 | 100,0 |
| Vergleichsbeispiel 8 | 0,49 | 10,93 | -- | -- | -- | 0,00 | 88,58 | 100,0 |
| Vergleichsbeispiel 9 | 0,49 | 10,93 | --- | -- | -- | 0,00 | 88,58 | 100,0 |
| Vergleichsbeispiel 10 | 0,49 | 10,93 | -- | -- | -- | 0,00 | 88,58 | 100,0 |
| Vergleichsbeispiel 11 | 0,49 | 10,93 | -- | -- | -- | 0,00 | 88,58 | 100,0 |
| Vergleichsbeispiel 12 | 0,49 | 10,93 | -- | | -- | 0,00 | 88,58 | 100,0 |

| | | | | | | | | |
|---|---|---|---|---|---|---|---|---|
| * Der genaue Gehalt an Bindemittel im kommerziell erhältlichen Vergleichsnagellack 18840 wurde nicht bestimmt. ** Die genauen Gehalte der Lösemittel des kommerziell erhältlichen Vergleichsnagellacks 18840 wurden nicht analysiert. Es wurden in dieser Tabelle bei den Vergleichsbeispielen 3 bis 12 nur die zugegebenen Gewichtsanteile an Ethylacetat und Butylacetat aufgeführt. Isopropanol wurde nicht zugegeben. | | | | | | | | |

### III Charakterisierung der optischen Eigenschaften der Nagellackkompositionen

### IIIa Bestimmung von Chroma (25° Geometrie)

Zur Bestimmung des optischen Erscheinungsbilds der erfindungsgemäßen Nagellackkompositionen sowie der Nagellackkompositionen der Vergleichsbeispiele wurde die jeweilige Nagellackkomposition mittels einer Spiralrakel (K Control Coater Modell 623, Fa. Erichsen) in einer Nassfilmschichtdicke von 100 µm auf Kontrastkartons appliziert und anschließend bei Raumtemperatur getrocknet. Von den so applizierten Nagellackkompositionen wurden als indirektes Maß des leafing-Verhaltens das Chroma (25° Geometrie) mit dem Gerät BYK Mac (Fa. BYK Gardner) vermessen.

Es wurden die Werte an mindestens 5 verschiedenen Stellen der Nagellackapplikation bestimmt. In nachstehender Tabelle 5 sind die hieraus gebildeten Mittelwerte für Chroma (25° Geometrie) aufgeführt.

Da diese farbmetrische Größe mit steigendem leafing Effekt der Pigmente ansteigen sollte, kann sie als indirektes Maß des leafing-Verhaltens der geprägten PVD-Pigmente angesehen werden.

### IIIb: Regenbogeneffekt und Brillanz:

Der Güte des Regenbogeneffektes wurde visuell nach einer Skala von 0 (kein Regenbogeneffekt) bis 10 (hervorragender Regenbogeneffekt) anhand der Rakelabzüge beurteilt. Mit den am Markt vorhandenen Farbmeßgeräten lassen sich die optischen Effekte von geprägten Effektpigmente, die auf Beugungsvorgängen beruhen, kaum sinnvoll messen, weshalb hier eine visuelle Beurteilung vorgezogen wurde.

Weiterhin wurde nach gleichem Notensystem ebenfalls die Brillanz beurteilt, bei der mit konventionellen Farbmeßgeräten gemessene Werte ebenfalls wenig aussagekräftig sein können.

**Tabelle 5: Ergebnisse des optischen Erscheinungsbildes aller Beispiele und Vergleichsbeispiele anhand von Rakelabzügen:**

| **Verwendete Pigmente** | **Vergleichsbeispiele (Nail Polish Base 18840)** | | | **Beispiele (Erfindungsgemäßer Nagellack)** | | |
|---|---|---|---|---|---|---|
| | **Regenbogen Effekt visuell** | **Brillanz visuell** | **Chroma 25° Farbmessung** | **RegenbogenEffekt visuell** | **Brillanz visuell** | **Chroma 25° Farbmessung** |
| Vergleichsbeispiel 1 | 1 | 2 | 11,7 | 0 | 0 | 1,1 |
| Beispiel 1 | 1 | 2 | 12,2 | 3 | 2 | 11,8 |
| Beispiel 2 | 1 | 2 | 13,2 | 3 | 2 | 15,8 |
| Beispiel 3 | 1 | 2 | 9,8 | 3 | 3 | 17,7 |
| Beispiel 4 | 1 | 2 | 10,5 | 2 | 2 | 11,6 |
| Beispiel 5 | 1 | 2 | 9,1 | 2 | 2 | 10,1 |
| Beispiel 6 | 1 | 2 | 9,4 | 3 | 3 | 16,7 |
| Vergleichsbeispiel 2 | 2 | 1 | 9,3 | 0 | 0 | 1,1 |
| Beispiel 7 | 2 | 1 | 8,4 | 8 | 8 | 44,7 |
| Beispiel 8 | 2 | 1 | 9,0 | 10 | 10 | 55,9 |
| Beispiel 9 | 2 | 1 | 9,5 | 6 | 6 | 32,8 |
| Beispiel 10 | 2 | 1 | 8,9 | 6 | 6 | 31,2 |

### IV Ergebnisse:

Alle erfindungsgemäßen Beispiele 1 bis 10 weisen gegenüber den entsprechenden Vergleichsbeispielen in dem kommerziell erhältlichen Nagellacksystem einen höheren Regenbogeneffekt auf. Besonders gravierend sind die Unterschiede bei dem geprägtem PVD-Pigment mit einem d₅₀-Wert von ca. 20 µm (Beispiele 7 bis 10). Bei diesen Beispielen sind ebenfalls deutliche Unterschiede in der Brillanz und im Chroma in den beiden Lacksystemen erkennbar. Bei dem gröberen geprägtem PVD-Pigment sind ebenfalls tendenziell höhere Chromawerte erkennbar, wenngleich in der Brillanz keine Verbesserungen erkennbar waren. Weiterhin zeigt sich, dass bei dem geprägtem PVD-Pigment mit einem d₅₀-Wert von ca. 20 µm mit Hostaphat CC100 (Cetylphosphorsäureester) beschichtete Beispiele im Vergleich zu mit Laurylphosphonsäure beschichteten Beispiele tendenziell am besten abschnitten (vergl. Beispiele 7 und 8 mit Beispielen 8 und 10). Bei den gröberen geprägten PVD-Pigmenten ist dieser Trend jedoch nicht eindeutig.

In allen Fällen sind die ohne Additivvorbehandlung verwendeten geprägten PVD-Pigmenten sowohl im Vergleichsnagellacksystem als auch in dem erfindungsgemäßen Nagellacksystem (Vergleichsbeispiele 1 und 2) schlecht hinsichtlich Regenbogeneffekt und Brillanz. Offenbar ist der durch die Additivbehandlung induzierte Leafing-Effekt der geprägten PVD-Pigmenten essentiell für das Entstehen eines ansprechenden Regenbogeneffekts.

## Patentansprüche

1. Nagellackkomposition enthaltend:
a) ein geprägtes Effektpigment umfassend ein plättchenförmiges metallisches Substrat mit geprägter Struktur, welche ein periodisches Muster mit diffraktiven Elementen aufweist und welches durch PVD-Verfahren hergestellt wird und optional wenigstens eine auf dem Substrat aufgebrachte Beschichtung, wobei das Substrat einen Gehalt an elementarem Metall von 80 bis 100 Gew.-%; bezogen auf das Substrat aufweist und wobei das Effektpigment mit einem leafing-Additiv zur Oberflächenmodifizierung behandelt wurde,
b) wenigstens ein Kohlenwasserstoffharz als Bindemittel,
c) wenigstens ein Lösemittel oder Lösemittelgemisch und
d) optional weitere Hilfsstoffe.

2. Nagellackkomposition nach Anspruch 1, wobei das leafing-Additiv der Gruppe bestehend aus Phosphorsäureestern der allgemeinen Formel:
(R-O)ₓ-P(O)(OR¹)₍₃₋ₓ₎ (I)
oder aus Phosphonsäureestern und/oder Phosphonsäuren der allgemeinen Formel
R-P(O)(OR²)(OR³), (II)
oder aus Fettsäuren der allgemeinen Formel
R'-COOH (III)
oder aus organofunktionellen Silanen der allgemeinen Formel
R"-Si(OR⁴)₃ (IV)
entnommen wird, wobei x = 1 bis 3 ist und die Reste R, R', R", R¹, R², R³ und R⁴ folgende Bedeutung haben:
R = linearer oder verzweigter Alkylrest mit einer Kohlenstoffkette aus einem Bereich von C₈ bis C₂₀;
R' = linearer oder verzweigter Alkylrest mit einer Kohlenstoffkette aus einem Bereich von C₁₂ bis C₂₆;
R" = linearer oder verzweigter Alkylrest mit einer Kohlenstoffkette aus einem Bereich von C₈ bis C₂₄;
R¹ = H, linearer und/oder verzweigter Alkylrest mit einer Kohlenstoffkette aus einem Bereich von C₁ bis C₆, bevorzugt C₁ bis C₃, wobei R¹ bei x = 1 identisch oder verschieden voneinander sein kann;
R² = R³ = H, linearer und/oder verzweigter Alkylrest mit einer Kohlenstoffkette aus einem Bereich von C₁ bis C₆, bevorzugt C₁ bis C₃, wobei R² und R³ identisch oder verschieden voneinander sein können und
R⁴ = linearer und/oder verzweigter Alkylrest mit einer Kohlenstoffkette aus einem Bereich von C₁ bis C₄, bevorzugt C₁ bis C₂.

3. Nagellackkomposition nach Anspruch 2, wobei das Additiv ein Phosphorsäureester gemäß Formel (I) mit R = C₁₂ bis C₁₈ ist und R¹ = H ist.

4. Nagellackkomposition nach einem der Ansprüche 2 oder 3, wobei als Additiv zur Oberflächenmodifizierung des Effektpigments jene der Gruppe bestehend aus Laurylphosphonsäuremethylester, Laurylphosphonsäureethylester, Laurylphosphonsäure (R = 12) oder Monocetylphosphorsäureester, Dicetylphosphorsäureester (R = 16) und Mischungen hiervon eingesetzt wird.

5. Nagellackkompositionen nach einem der vorstehenden Ansprüche, wobei das plättchenförmige, metallische Substrat mit geprägter Struktur 5.000 bis 20.000 diffraktive Elemente/cm aufweist.

6. Nagellackkompositionen nach einem der vorstehenden Ansprüche, wobei das plättchenförmige, metallische Substrat mit geprägter Struktur ein Metall aus der Gruppe bestehend aus Aluminium, Kupfer, Chrom, Eisen oder Legierungen hiervon, entnommen ist.

7. Nagellackkompositionen nach Anspruch 6, wobei das plättchenförmige, metallische Substrat mit geprägter Struktur Aluminium ist.

8. Nagellackkomposition nach einem der vorstehenden Ansprüche, wobei das Kohlenwasserstoffharz der Gruppe bestehend aus Cumaron-Inden-Harzen, Polystyrolhaltigen Harze, durch Polykondensation von Formaldehyd-Harzen abgeleitete Harze und deren Mischungen, entnommen wird.

9. Nagellackkomposition nach Anspruch 8, wobei das Kohlenwasserstoffharz ein Polystyrol-haltiges Harz ist, welches überwiegend bis gänzlich durch Polymerisation verschiedener Styrolmonomere, insbesondere optisch weitgehend transparente Styrolmonomere, gewonnen werden.

10. Nagellackkomposition nach einem der vorstehenden Ansprüche, wobei das Kohlenwasserstoffharz 80 bis 100 Gew.-% des gesamten organischen Bindemittels ausmacht.

11. Nagellackkompositionen nach einem der vorstehenden Ansprüche, wobei der Gehalt an Kohlenwasserstoffharzen in einem Bereich von 25 Gew.-% bis 63 Gew.-%, bezogen auf die gesamte Nagellackkomposition, liegt.

12. Nagellackkompositionen nach einem der vorstehenden Ansprüche, wobei das Kohlenwasserstoffharz aus wenigstens zwei voneinander verschiedene Kohlenwasserstoffharze mit einem ersten mittleren Molekulargewicht Mw aus einem Bereich von 1.000 bis 2.000 g/mol und einem zweiten mittleren Molekulargewicht Mw aus einem Bereich von 4.000 bis 5.900 g/mol im Gewichtsverhältnis 1:1 bis 1:10 besteht.

13. Nagellackkompositionen nach Anspruch 12, wobei das Kohlenwasserstoffharz aus wenigstens zwei voneinander verschiedenen Kohlenwasserstoffharzen mit einem mittleren Molekulargewicht Mw aus einem Bereich von 1.200 bis 1.600 g/mol und einem mittleren Molekulargewicht Mw aus einem Bereich von 4.500 bis 5.500 g/mol im Gewichtsverhältnis 1:1 bis 1:10 besteht.

14. Nagellackkomposition nach einem der vorstehenden Ansprüche, enthaltend als Lösemittel eine Mischung aus Isopropanol, Ethylacetat und Butylacetat, wobei die Lösemittelmischung aus Isopropanol, Ethylacetat und Butylacetat 70 bis 100 Gew.-% des gesamten Lösemittels ausmacht.

15. Verfahren zur Herstellung der Nagellackkomposition nach den Ansprüchen 1 bis 14, umfassend die Schritte
i) Oberflächenmodifizierung des geprägten Effektpigments durch ein leafing-Additiv in einer Dispersion in einem Lösemittel,
ii) Lösen des Kohlenwasserstoffharz in einem Lösemittel oder Lösemittelgemisch
iii) Vermischen und Homogenisieren der Dispersion nach i) mit der Bindemittellösung nach ii), sowie
iv) gegebenenfalls Ergänzen mit weiterem Lösemittel oder Lösemittelgemisch.

## Claims

1. Nail varnish composition comprising:
a) an embossed effect pigment comprising a metallic substrate in platelet form with embossed structure having a periodic pattern with diffractive elements, said substrate having been produced by PVD methods, and optionally at least one coating applied to the substrate, wherein the substrate has an elemental metal content of 80% to 100% by weight, based on the substrate, and wherein the effect pigment has been treated with a leafing additive for surface modification,
b) at least one hydrocarbon resin as binder,
c) at least one solvent or solvent mixture and
d) optionally further auxiliaries.

2. Nail varnish composition according to Claim 1, wherein the leafing additive is taken from the group consisting of phosphoric esters of the general formula:
(R-O) ₓ-P (O) (OR¹) ₍₃₋ₓ₎ (I)
or from phosphonic esters and/or phosphonic acids of the general formula
R-P(O) (OR²) (OR³) (II)
or from fatty acids of the general formula
R'-COOH (III)
or from organofunctional silanes of the general formula
R"-Si(OR⁴)₃ (IV)
where x = 1 to 3 and the R, R", R", R¹, R², R³ and R⁴ radicals are defined as follows:
R = linear or branched alkyl radical having a carbon chain from a range from C₈ to C₂₀;
R' = linear or branched alkyl radical having a carbon chain from a range from C₁₂ to C₂₆;
R" = linear or branched alkyl radical having a carbon chain from a range from C₈ to C₂₄;
R¹ = H, linear and/or branched alkyl radical having a carbon chain from a range from C₁ to C₆, preferably C₁ to C₃, where R¹ when x = 1 may be identical or different;
R² = R³ = H, linear and/or branched alkyl radical having a carbon chain from a range from C₁ to C₆, preferably C₁ to C₃, where R² and R³ may be identical or different, and
R⁴ = linear and/or branched alkyl radical having a carbon chain from a range from C₁ to C₄, preferably C₁ to C₂.

3. Nail varnish composition according to Claim 2, wherein the additive is a phosphoric ester of formula (I) with R = C₁₂ to C₁₈ and R¹ = H.

4. Nail varnish composition according to either of Claims 2 and 3, wherein the additive used for surface modification of the effect pigment is one from the group consisting of laurylphosphonic acid methyl ester, laurylphosphonic acid ethyl ester, laurylphosphonic acid (R = 12) or monocetylphosphoric ester, dicetylphosphoric ester (R = 16) and mixtures thereof.

5. Nail varnish compositions according to any of the preceding claims, wherein the metallic substrate in platelet form with an embossed structure has 5000 to 20 000 diffractive elements/cm.

6. Nail varnish compositions according to any of the preceding claims, wherein the metallic substrate in platelet form with embossed structure is a metal taken from the group consisting of aluminum, copper, chromium, iron and alloys thereof.

7. Nail varnish compositions according to Claim 6, wherein the metallic substrate in platelet form with embossed structure is aluminum.

8. Nail varnish composition according to any of the preceding claims, wherein the hydrocarbon resin is taken from the group consisting of coumarone-indene resins, polystyrene-containing resins, resins derived from formaldehyde resins by polycondensation, and mixtures thereof.

9. Nail varnish composition according to Claim 8, wherein the hydrocarbon resin is a polystyrene-containing resin which is obtained predominantly to entirely by polymerization of different styrene monomers, especially largely optically transparent styrene monomers.

10. Nail varnish composition according to any of the preceding claims, wherein the hydrocarbon resin accounts for 80% to 100% by weight of the overall organic binder.

11. Nail varnish compositions according to any of the preceding claims, wherein the content of hydrocarbon resins is within a range from 25% by weight to 63% by weight, based on the overall nail varnish composition.

12. Nail varnish compositions according to any of the preceding claims, wherein the hydrocarbon resin consists of at least two different hydrocarbon resins having a first average molecular weight Mw from a range from 1000 to 2000 g/mol and a second average molecular weight M_{w} from a range from 4000 to 5900 g/mol in a weight ratio of 1:1 to 1:10.

13. Nail varnish compositions according to claim 12, wherein the hydrocarbon resin consists of at least two different hydrocarbon resins having an average molecular weight M_{w} from a range from 1200 to 1600 g/mol and an average molecular weight Mw from a range from 4500 to 5500 g/mol in a weight ratio of 1:1 to 1:10.

14. Nail varnish composition according to any of the preceding claims, comprising, as solvent, a mixture of isopropanol, ethyl acetate and butyl acetate, wherein the solvent mixture of isopropanol, ethyl acetate and butyl acetate accounts for 70% to 100% by weight of the overall solvent.

15. Process for producing the nail varnish composition according to any of Claims 1 to 14, comprising the steps of
i) surface-modifying the embossed effect pigment by means of a leafing additive in a dispersion in a solvent,
ii) dissolving the hydrocarbon resin in a solvent or solvent mixture,
iii) mixing and homogenizing the dispersion according to i) with the binder solution according to ii), and
iv) optionally supplementing with further solvent or solvent mixture.

## Revendications

1. Composition de vernis à ongles contenant !
a) un pigment à effet gaufré comprenant un substrat métallique en paillettes à structure gaufrée qui présente un motif périodique à éléments diffractifs, et qui est produit par un procédé PVD et comporte en option au moins un revêtement appliqué sur le substrat, le substrat ayant une teneur en métal élémentaire de 80 à 100 % en poids, par rapport au substrat et le pigment à effet ayant été traité par un additif de feuilletage destiné à la modification de la surface,
b) au moins une résine hydrocarbonée en tant que liant,
c) au moins un solvant ou mélange de solvants et
d) en option d'autres adjuvants.

2. Composition de vernis à ongles selon la revendication 1, dans laquelle l'additif de feuilletage est choisi dans le groupe constitué par les esters d'acide phosphorique de formule générale :
(R-O)ₓ-P(O) (OR¹) ₍₃₋ₓ₎ (I)
ou par les esters d'acides phosphoniques et/ou les acides phosphoniques de formule générale
R-P(O) (OR²) (OR³) , (II)
ou par les acides gras de formule générale
R'-COOH (III)
ou par les silanes organofonctionnels de formule générale
R"-Si (OR⁴)₃ (IV)
où x = 1 à 3 et les radicaux R, R', R", R¹, R², R³ et R⁴ ont les significations suivantes :
R = un radical alkyle linéaire ou ramifié ayant une chaîne carbonée dans une plage de C₈ à C₂₀ ;
R' = un radical alkyle linéaire ou ramifié ayant une chaîne carbonée dans une plage de C₁₂ à C₂₆ ;
R" = un radical alkyle linéaire ou ramifié ayant une chaîne carbonée dans une plage de C₈ à C₂₄ ;
R¹ = H, un radical alkyle linéaire et/ou ramifié ayant une chaîne carbonée dans une plage de C₁ à C₆, de préférence C₁ à C₃, R¹ lorsque x = 1 pouvant être le même ou différent dans un cas et dans l'autre ;
R² = R³ = H, un radical alkyle linéaire et/ou ramifié ayant une chaîne carbonée dans une plage de C₁ à C₆, de préférence C₁ à C₃, R² et R³ pouvant être identiques ou différents l'un de l'autre et
R⁴ = un radical alkyle linéaire et/ou ramifié ayant une chaîne carbonée dans une plage de C₁ à C₄, de préférence C₁ à C₂.

3. Composition de vernis à ongles selon la revendication 2, dans laquelle l'additif est un ester d'acide phosphorique selon la formule (1) où R est en C₁₂ à C₁₈ et R¹ = H.

4. Composition de vernis à ongles selon l'une quelconque des revendications 2 et 3, dans laquelle on utilise comme additif destiné à la modification de la surface du pigment à effet l'un du groupe constitué par le laurylphosphonate de méthyle, le laurylphosphonate d'éthyle, l'acide laurylphosphonique (R = 12) ou le phosphate de monocétyle, le phosphate de dicétyle (R = 16) et des mélanges de ceux-ci.

5. Compositions de vernis à ongles selon l'une quelconque des revendications précédentes, dans lesquelles le substrat métallique en paillettes à structure gaufrée comporte 5 000 à 20 000 éléments diffractifs.

6. Compositions de vernis à ongles selon l'une quelconque des revendications précédentes, dans lesquelles le substrat métallique en paillettes à structure gaufrée est un métal choisi dans le groupe constitué par l'aluminium, le cuivre, le chrome, le fer ou des alliages de ceux-ci.

7. Compositions de vernis à ongles selon la revendication 6, dans lesquelles le substrat métallique en paillettes à structure gaufrée est l'aluminium.

8. Composition de vernis à ongles selon l'une quelconque des revendications précédentes, dans laquelle la résine hydrocarbonée est choisie dans le groupe constitué par les résines coumarone-indène, les résines contenant du polystyrène, les résines obtenues par polycondensation de résines formaldéhyde et des mélanges de celles-ci.

9. Composition de vernis à ongles selon la revendication 8, dans laquelle la résine hydrocarbonée est une résine contenant du polystyrène, qui est obtenue principalement à totalement par polymérisation de divers monomères styrène, en particulier monomères styrène essentiellement optiquement transparents.

10. Composition de vernis à ongles selon l'une quelconque des revendications précédentes, dans laquelle la résine hydrocarbonée constitue 80 à 100 % en poids du liant organique total.

11. Compositions de vernis à ongles selon l'une quelconque des revendications précédentes, dans lesquelles la teneur en résines hydrocarbonées se situe dans une plage de 25 % en poids à 63 % en poids, par rapport à la composition de vernis à ongles totale.

12. Compositions de vernis à ongles selon l'une quelconque des revendications précédentes, dans lesquelles la résine hydrocarbonée consiste en au moins deux résines hydrocarbonées différentes l'une de l'autre, ayant une première masse moléculaire moyenne M_{w} dans une plage de 1 000 à 2 000 g/mole et une seconde masse moléculaire moyenne M_{w} dans une plage de 4 000 à 5 900 g/mole en un rapport pondéral de 1:1 à 1:10.

13. Compositions de vernis à ongles selon la revendication 12, dans lesquelles la résine hydrocarbonée consiste en au moins deux résines hydrocarbonées différentes l'une de l'autre, ayant une masse moléculaire moyenne M_{w} dans une plage de 1 200 à 1 600 g/mole et une masse moléculaire moyenne M_{w} dans une plage de 4 500 à 5 500 g/mole en un rapport pondéral de 1:1 à 1:10.

14. Composition de vernis à ongles selon l'une quelconque des revendications précédentes, contenant en tant que solvant un mélange d'isopropanol, acétate d'éthyle et acétate de butyle, le mélange de solvants constitué d'isopropanol, acétate d'éthyle et acétate de butyle constituant 70 à 100 % en poids du solvant total.

15. Procédé pour la préparation de la composition de vernis à ongles selon les revendications 1 à 14, comprenant les étapes
i) modification de la surface du pigment à effet gaufré, par un additif de feuilletage en une dispersion dans un solvant,
ii) dissolution de la résine hydrocarbonée dans un solvant ou mélange de solvants
iii) mélange et homogénéisation de la dispersion selon i) avec la solution de liant selon ii), ainsi que
iv) éventuellement complément avec un autre solvant ou mélange de solvants.
